(19) Europäisches Patentamt
European Patent Office
Office européen des brevets

(11) EP 0 491 263 B1

(12) EUROPÄISCHE PATENTSCHRIFT

(45) Veröffentlichungstag und Bekanntmachung des Hinweises auf die Patenterteilung:
**24.07.1996 Patentblatt 1996/30**

(51) Int. Cl.[6]: **C07C 217/12**, C07C 217/60, C07C 217/08, C07C 323/25, C07C 211/27

(21) Anmeldenummer: **91121136.5**

(22) Anmeldetag: **10.12.1991**

(54) **Phenylalkylaminoalkyl-Verbindungen sowie Verfahren zu ihrer Herstellung und diese Verbindungen enthaltende Arzneimittel**

Phenylalkylaminoalkyl compounds; process for their preparation and pharmaceutical compositions containing same

Composés phénylalkylaminoalkyliques, procédé pour leur préparation et compositions pharmaceutiques qui les contiennent

(84) Benannte Vertragsstaaten:
**AT BE CH DE DK ES FR GB GR IT LI LU NL SE**

(30) Priorität: **19.12.1990 DE 4040632**

(43) Veröffentlichungstag der Anmeldung:
**24.06.1992 Patentblatt 1992/26**

(73) Patentinhaber: **Kali-Chemie Pharma GmbH**
**D-30173 Hannover (DE)**

(72) Erfinder:
- **Hell, Insa**
  **W-3000 Hannover 1 (DE)**
- **Preuschoff, Ulf**
  **W-3014 Laatzen (DE)**
- **Krähling, Hermann**
  **W-3163 Sehnde 1 (DE)**
- **David, Samuel**
  **W-3000 Hannover 1 (DE)**
- **Ban, Ivan**
  **W-3000 Hannover 72 (DE)**
- **Christen, Marie-Odile**
  **F-75016 Paris (FR)**

(74) Vertreter: **Lauer, Dieter, Dr.**
**Solvay Pharma Deutschland GmbH**
**Hans-Böckler-Allee 20**
**D-30173 Hannover (DE)**

(56) Entgegenhaltungen:
**CH-A- 471 075**
**FR-A- 1 260 571**
**US-A- 3 703 554**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 491 263 B1

Printed by Rank Xerox (UK) Business Services
2.13.0/3.4

**Beschreibung**

Die vorliegende Erfindung betrifft neue Dialkylamin-Derivate, welche einen durch eine einen mono- oder bicyclischen Kohlenwasserstoffrest enthaltende Alkoxy-, Alkylthio- oder Alkylaminogruppe substituierten Alkylrest und einen ein gegebenenfalls substituiertes Phenyl tragenden Alkylrest enthalten, sowie diese Verbindungen enthaltende pharmazeutische Zubereitungen und Verfahren zur Herstellung dieser Verbindungen.

Der vorliegenden Erfindung liegt die Aufgabe zugrunde, neue Dialkylamino-Verbindungen mit wertvollen pharmakologischen Eigenschaften herzustellen.

Es wurde nun gefunden, daß die neuen Phenylalkylaminoalkyl-Verbindungen, welche am Alkylrest über Sauerstoff, Schwefel oder eine Alkylaminogruppe gebundene mono- oder bicyclische, von Terpenen abgeleitete Kohlenwasserstoffreste enthalten, wertvolle pharmakologische Eigenschaften besitzen und insbesondere eine günstige pharmakologische Wirkung im gastrointestinalen Trakt zeigen. Sie zeichnen sich insbesondere durch die gastrointestinale Schleimhaut schützende und ulkushemmende Wirkungen bei guter Verträglichkeit und geringer Toxizität aus und besitzen daneben auch gastrointestinal wirksame spasmolytische Eigenschaften.

In dem Schweizer Patent Nr. 471 075 werden eine substituierte Aminoalkylgruppe enthaltende basische Terpenäther-Derivate mit einer starken entzündungshemmenden Wirkung beschrieben. Der erfindungsgemäßen Verbindungen fallen teilweise unter die in dem Schweizer Patent angegebene allgemeine Formel, sind jedoch in dem Schweizer Patent selbst nicht beschrieben oder genannt.

Die erfindungsgemäßen Verbindungen unterscheiden sich in ihrer chemischen Struktur von den in dem Schweizer Patent beschriebenen Verbindungen dadurch, daß der Substituent der Aminogruppe eine Phenylgruppe enthält, während die substituierte Aminogruppe der in dem Schweizer Patent beschriebenen Verbindungen eine Dialkylamino- oder Morpholinogruppe darstellt.

Diese vorbekannten Verbindungen sind pharmakologisch zu den nichtsteroidalen Entzündungshemmern (=NSAID = nonsteroidal anti-inflammatory drugs) zu rechnen. Von NSAIDs ist allgemein bekannt, daß sie zu ulcusfördernden Nebenwirkungen im gastrointestinalen neigen. Die erfindungsgemäßen Verbindungen besitzen keine entzündungshemmende Wirkung, zeichnen sich jedoch überraschenderweise durch gastrointestinale protektive ulcushemmende Wirkungen aus.

Die vorliegende Erfindung betrifft daher neue Phenylalkylaminoalkyl-Verbindungen der allgemeinen Formel I

(siehe Formel I)

worin

| | |
|---|---|
| m | für 1-4 steht, |
| n | für 2-5 steht, |
| $R^1$ | Wasserstoff oder Alkyl mit 1 - 4 Kohlenstoffatomen bedeutet, |
| $R^2$ | Wasserstoff, Alkyl mit 1 - 4 Kohlenstoffatomen, Alkoxy mit 1 - 4 Kohlenstoffatomen, Halogen oder Trifluormethyl bedeutet, und |
| $R^3$ | Wasserstoff, Alkyl mit 1 - 4 Kohlenstoffatomen, Alkoxy mit 1 - 4 Kohlenstoffatomen oder Halogen bedeutet, oder |
| $R^2$ und $R^3$ | an benachbarte Kohlenstoffatome gebunden sind und gemeinsam eine Alkylendioxygruppe mit 1-2 Kohlenstoffatomen bedeuten, und |
| $R^4$ | für einen gesättigten mono- oder bicyclischen Terpenkohlenwasserstoffrest mit 10 Kohlenstoffatomen oder für einen bicyclischen Kohlenwasserstoffrest mit 11 Kohlenstoffatomen mit der Formel a (siehe Formel a) steht, und |
| Z | für Sauerstoff, eine Gruppe N-$R^5$, worin $R^5$ niederes Alkyl mit 1 - 4 Kohlenstoffatomen bedeutet, oder, falls $R^4$ eine Gruppe der Formel a bedeutet, auch für Schwefel steht, |

und deren physiologisch verträgliche Säureadditionssalze.

In den Verbindungen der Formel I kann $R^1$ Wasserstoff oder vorzugsweise niederes Alkyl bedeuten. Eine niedere Alkylgruppe $R^1$ kann geradkettig oder verzweigt sein und 1 bis 4 Kohlenstoffatome enthalten und stellt insbesondere die Methylgruppe dar.

Sofern die Substituenten $R^2$ und/oder $R^3$ der Phenylgruppe niedere Alkyl- oder Alkoxygruppen darstellen, können diese geradkettig oder verzweigt sein und 1 bis 4, insbesondere 1 bis 2, Kohlenstoffatome enthalten und stellen vorzugsweise Methyl oder Methoxy dar. Als Halogensubstituenten der Phenylgruppe kommen insbesondere Fluor, Chlor oder Brom in Frage.

Die Alkylenkette $-(CH_2)_m-$ kann 1 bis 4, vorzugsweise 1 bis 2, insbesondere 2, Glieder enthalten. Die Alkylenkette $-(CH_2)_n-$ kann 2 bis 5, vorzugsweise 2 oder 3, Glieder enthalten. Z steht vorzugsweise für Sauerstoff.

Die Kohlenwasserstoff-Reste $R^4$ der Verbindungen der Formel I sind von Terpenen abgeleitete Reste mit 10 oder 11 Kohlenstoffatomen, welche 17 - 19 Wasserstoffatome enthalten können. In den Resten sind jeweils mehrere Chiralitätszentren enthalten, welche R- oder S-konfiguriert sein können, sodaß die Substanzen in mehreren diastereoisomeren Formen auftreten können. Erfindungsgemäß umfassen die Verbindungen der Formel I die einzelnen stereoisomeren Formen der Verbindungen der Formel I und deren Gemische. Bevorzugt enthalten die Verbindungen der Formel I solche Kohlenwasserstoff-Reste $R^4$, welche sich von natürlich vorkommenden Terpenderivaten ableiten, beziehungsweise sich aus diesen herstellen lassen. In der Natur kommen Terpenderivate, z.B. gesättigte Terpenalkohole, als Pflanzeninhaltsstoffe in mehr oder weniger sterisch reiner Form oder als Stereoisomerengemische wechselnder Zusammensetzung vor. Dementsprechend liegen auch käuflich erhältliche Terpenderivate, welche im allgemeinen aus Naturprodukten hergestellt werden, in mehr oder weniger sterisch reiner Form oder als Stereoisomerengemische vor. Im Rahmen der vorliegenden Erfindung kommen insbesondere solche Verbindungen der Formel I in Betracht, in welchen der Kohlenwasserstoff-Rest $R^4$ von natürlichen und/oder käuflich erhältlichen Terpenderivaten abstammt.

Bei der erfindungsgemäßen Herstellung von Verbindungen der Formel I bleibt die Konfiguration des Kohlenwasserstoff-Restes $R^4$ der Ausgangsverbindungen $R^4$-Z-H erhalten, so daß je nach eingesetztem Ausgangsprodukt als Endprodukte der Formel I Stereoisomerengemische oder mehr oder weniger stereoisomerenreine Substanzen der Formel I erhalten werden.

Als Kohlenwasserstoff-Reste $R^4$ eignen sich insbesondere mono- oder bicyclische Kohlenwasserstoffreste mit 10 oder 11 Kohlenstoffatomen aus der Gruppe 2-(6,6-Dimethylbicyclo[3.1.1]hept-2-yl)-ethyl (=Dihydronopyl) der Formel a, 1-Methyl-4-isopropylcyclohex-3-yl (= Menthyl) der Formel b, 6,6-Dimethylbicyclo[3.1.1]hept-2-ylmethyl (= Myrtanyl) der Formel c oder auch 1,3,3-Trimethylbicyclo[2.2.1]hept-2-yl (= Fenchyl) der Formel d und 1,7,7-Trimethylbicyclo[2.2.1]hept-2-yl (= Bornyl) der Formel e

(siehe Formeln a, b, c, d und e)

Unter den vorgenannten Resten $R^4$ ist insbesondere der 2-(6,6-Dimethylbicyclo[3.1.1]hept-2-yl)-ethyl-Rest der Formel a (= Dihydronopylrest) gut geeignet. Dieser Rest enthält in den Positionen 1, 2 und 5 des Bicycloheptan-Ringgerüstes Chiralitätszentren, welche jeweils R-oder S-konfiguriert sein können. Im Rahmen der vorliegenden Erfindung liegt in den Dihydronopyl-Verbindungen der Formel I bevorzugt eine 2-(6,6-Dimethylbicyclo[3.1.1]hept-2-yl)-ethyl-Gruppierung vor, welche von einem Terpenalkohol stammt, welcher sich von dem natürlichen (-)-β-Pinen (= (1S,5S)-(-)-6,6-Dimethyl-2-methylenbicyclo[3,1,1]heptan) ableitet, in welchem die Chiralitätszentren in 1- und 5-Position S-Konfiguration besitzen. Dementsprechend sind auch in den 2-(6,6-Dimethylbicyclo[3.1.1]hept-2-yl)-ethyl-Derivaten der Formel I die Chiralitätszentren in 1- und 5-Position des Terpenringgerüstes S-konfiguriert, während das Chiralitätszentrum in 2-Position S- oder R-Konfiguration besitzen kann, sodaß die entsprechenden Substanzen der Formel I in zwei diastereomeren Formen auftreten können. Von diesen beiden Dihydronopyl-Formen werden insbesondere die cis-Form, in welcher das Chiralitätszentrum in Position 2 des Bicycloheptan-Ringgerüstes S-Konfiguration besitzt, oder Gemische, in denen diese Form in überwiegendem Maße enthalten ist, eingesetzt.

Ferner ist als Kohlenwasserstoffrest $R^4$ der 6,6-Dimethylbicyclo[3.1.1]hept-2-ylmethyl-Rest der Formel c (= Myrtanylrest) gut geeignet. Dieser Rest enthält in den Positionen 1, 2 und 5 des Bicycloheptan-Ringgerüstes Chiralitätszentren, welche jeweils R- oder S-konfiguriert sein können. Im Rahmen der vorliegenden Erfindung liegt in den Myrtanyl-Verbindungen der Formel I ebenfalls bevorzugt eine 6,6-Dimethylbicyclo[3.1.1]hept-2-ylmethyl-Gruppierung vor, welche sich von dem natürlichen (-)-β-Pinen (=(1S,5S)-(-)-6,6-Dimethyl-2-methylenbicyclo[3.1.1]heptan) ableitet, in welchem die Chiralitätszentren in 1- und 5-Position S-Konfiguration besitzen. Dementsprechend sind auch in den Myrtanyl-Derivaten der Formel I die Chiralitätszentren in 1- und 5-Position des Bicycloheptangerüstes bevorzugt S-konfiguriert, während das Chiralitätszentrum in 2-Position S- oder R-Konfiguration besitzen kann. Insbesondere werden cis-Myrtanyl-Derivate, in welchen alle drei Chiralitätszentren des Bicycloheptan-Ringgerüstes S-Konfiguration besitzen, oder Gemische, in denen diese Form in überwiegendem Maße enthalten ist, eingesetzt.

Als Rest $R^4$ eignet sich ferner der 1-Methyl-4-isopropylcyclohex-3-yl-Rest der Formel b (= Menthylrest). Dieser von Menthol abstammende Rest enthält in den Positionen 1, 3 und 4 des Cyclohexylgerüstes Chiralitätszentren, welche jeweils R- oder S-konfiguriert sein können. In der Natur kommen am häufigsten die beiden enantiomeren Formen 1R,3R,4S-1-Methyl-4-isopropylcyclohexan-3-ol (= L-Menthol) und 1S,3S,4R-1-Methyl-4-isopropylcyclohexan-3-ol (= D-Menthol) vor. Auch im Rahmen der vorliegenden Erfindung sind unter den menthylsubstituierten Verbindungen der Formel I diese beiden Menthylformen, insbesondere die L-Menthylform oder Stereoisomerengemische, in denen die L-Menthylform überwiegt, bevorzugt.

Als Kohlenwasserstoffreste $R^4$ eignen sich auch der 1,3,3-Trimethylbicyclo[2.2.1]hept-2-yl-Rest der Formel d (= Fenchylrest) und der 1,7,7-Trimethylbicyclo[2.2.1]hept-2-yl-Rest der Formel e (= Bornylrest).

In dem Fenchylrest $R^4$ sind in den Positionen 1, 2 und 4 des Bicycloheptan-Ringgerüstes Chiralitätszentren, welche jeweils R- oder S-konfiguriert sein können, enthalten. Im Rahmen der vorliegenden Erfindung sind Fenchyl-Reste bevorzugt, welche sich von natürlichem (+)-Fenchol ableiten, in welchem eine 1S,4R-Konfiguration vorliegt, während das Chiralitätszentrum in 2-Position vorzugsweise R-Konfiguration aber auch S-Konfiguration besitzen kann.

Der Bornyl-Rest $R^4$ enthält in den Positionen 1, 2 und 4 des Bicycloheptan-Ringgerüstes Chiralitätszentren, welche jeweils R- oder S-konfiguriert sein können. In natürlichen Bornyl-Derivaten kommt hauptsächlich der Endobornyl-Rest vor. Es überwiegt die (-)-Form (= 1S,2R,4S-Form) gegenüber der (+)-Form (= 1R,2S,4R-Form). Auch im Rahmen der

vorliegenden Erfindung werden Bornyl-Derivate bevorzugt, deren Bornyl-Rest von natürlichem Borneol abstammt und in der 1S,2R,4S-Form vorliegt, oder Gemische, in denen diese Form in überwiegendem Maße enthalten ist.

Die neuen Phenylalkylaminoalkyl-Verbindungen der Formel I besitzen wertvolle pharmakologische Eigenschaften, insbesondere eine günstige pharmakologische Wirkung im gastrointestinalen Trakt. Sie zeichnen sich durch eine Schutzwirkung auf die gastrointestinale Schleimhaut und durch eine Hemmwirkung auf Ulkusbildung bei einer guten Verträglichkeit und nur geringer Toxizität aus. Bekanntermaßen üben die nicht steroidalen Antiphlogistika eine schädigende Wirkung auf die Mucosabarriere aus, und gastrotoxische Dosen derartiger Arzneimittel oder anderer Chemikalien, z.B. Alkohol, können zu Läsionen und Ulkusbildung führen. Dieser Effekt läßt sich durch die erfindungsgemäßen neuen Verbindungen hemmen, was auf eine therapeutisch protektive Wirkung an der gastrointestinalen Schleimhaut hinweist.

Diese Wirkungen lassen sich in pharmakologischen Standardtests an Tieren nachweisen.

Beschreibung der pharmakologischen Versuche:

A. Bestimmung der Hemmwirkung der Verbindungen gegen durch Ethanol induzierte Läsionen in Ratten.

Es werden pro Testdosis Gruppen von 6 männlichen Ratten mit einem Körpergewicht von 160-180 g verwendet. Die Tiere werden 24 Stunden nüchtern gehalten, wobei ihnen Wasser unbegrenzt zur Verfügung steht. Die Prüfsubstanzen werden suspendiert in 0,5 ml Suspensionsmedium pro 100 g Tiergewicht per os appliziert. Eine Kontrollgruppe von Tieren erhält nur das entsprechende Volumen an Suspensionsmedium. Eine Stunde nach Applikation der Testsubstanzen werden den Tieren per os 0,5 ml 60%-iges Ethanol pro 100 g Tiergewicht verabreicht. Die Tiere werden 1 Stunde nach der Ethanolapplikation getötet. Die Mägen werden entnommen, geöffnet und vorsichtig abgespült. Anzahl und Größe der in der Schleimhaut gebildeten Läsionen werden beurteilt. Die Auswertung erfolgt modifiziert nach O. Münchow (Arzneim. Forsch. (Drug Res.) 4, 341 - 344 (1954)). Es werden Mittelwerte und Standardabweichungen berechnet und daraus die Hemmwirkung der Prüfsubstanzen in % gegenüber der Kontrollgruppe bestimmt.

Die nachfolgende Tabelle A gibt nach der vorstehend beschriebenen Testmethode erhaltene Ergebnisse wieder. Die für die Verbindungen der Formel I angegebenen Beispielsnummern beziehen sich auf die nachstehenden Herstellungsbeispiele.

Tabelle A

| Beispiel Nr. | Dosis in µmol/kg p.o | Hemmwirkung auf Ethanolinduzierte Läsionen im Rattenmagen % Hemmung |
|---|---|---|
| 1 | 100 | 77 |
| 2 | 100 | 93 |
| 5 | 100 | 97 |
| | 46 | 87 |
| 6 | 46 | 59 |
| 7 | 100 | 61 |
| 11 | 100 | 72 |
| 12 | 100 | 86 |
| | 46 | 62 |
| 13 | 100 | 87 |
| | 46 | 72 |
| 18 | 100 | 75 |
| 20 | 100 | 83 |
| | 46 | 63 |
| 28 | 100 | 63 |

B. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird als minimale toxische Dosis in der nachfolgenden Tabelle B angegeben.

Tabelle B

| Beispiel Nr. | minimale toxische Dosis in der Maus mg/kg p.o |
|---|---|
| 1 | 100 |
| 5 | 300 |
| 6 | 300 |
| 7 | > 300 |
| 11 | 300 |
| 12 | > 300 |
| 13 | > 300 |
| 18 | > 300 |
| 20 | 300 |
| 28 | 300 |

Neben einer Schutzwirkung auf die gastrointestinale Schleimhaut und einer ulkushemmenden Wirkung besitzen die Verbindungen der Formel I auch gastrointestinal wirksame spasmolytische Eigenschaften.

Aufgrund ihrer Wirkungen im gastrointestinalen Trakt sind die Verbindungen der Formel I in der Gastroenterologie als Arzneimittel für größere Säugetiere, insbesondere Menschen, geeignet zur Prophylaxe und Behandlung von Ulkusbildung und von durch gastrotoxische Dosen von Arzneimitteln oder Chemikalien hervorgerufenen Schädigungen der gastrointestinalen Mucosa.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 10 bis 200 mg pro Einzeldosis.

Als Heilmittel können die Verbindungen der Formel I mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägersstoffe wie z.B. Milchzucker, Stärke oder Talkum oder flüssigen Paraffinen und unter Verwendung von pharmazeutisch üblichen Hilfsstoffen, beispielsweise Tablettensprengmitteln, Lösungsvermittlern oder Konservierungsmitteln.

Erfindungsgemäß werden die neuen Verbindungen der Formel I und deren Säureadditionssalze erhalten, indem man in an sich bekannter Weise

a) Verbindungen der allgemeinen Formel II

(siehe Formel II)

worin $R^2$, $R^3$ und m obige Bedeutung besitzen und Q für den Rest

```
   R1
  /
-N   ,
  \
   H
```

worin $R^1$ obige Bedeutung besitzt, steht, oder falls m nicht 2 bedeutet, auch für eine abspaltbare Fluchtgruppe Y steht, umsetzt mit Verbindungen der allgemeinen Formel III

(siehe Formel III)

worin $R^4$, Z und n obige Bedeutung besitzen und Q' für eine abspaltbare Fluchtgruppe Y steht, falls Q den Rest

```
   R1
  /
-N
  \
   H
```

bedeutet, oder Q' für den Rest

```
   R1
  /
-N
  \
   H
```

steht, falls Q eine abspaltbare Fluchtgruppe Y bedeutet, oder

b) Verbindungen der allgemeinen Formel IV

(siehe Formel IV)

worin $R^1$, $R^2$, $R^3$, $R^4$, Z, n und m obige Bedeutung besitzen, reduziert, oder

c) zur Herstellung von Verbindungen der allgemeinen Formel Ia

(siehe Formel Ia)

worin $R^2$, $R^3$, $R^4$ und n obige Bedeutung besitzen, m' 2-4 bedeutet und Z' die für Z angegebene Bedeutung mit Ausnahme von Schwefel besitzt, aus Verbindungen der allgemeinen Formel V

(siehe Formel V)

worin $R^2$, $R^3$, $R^4$, Z', n und m' obige Bedeutung besitzen, und B für eine hydrogenolytisch abspaltbare Gruppe steht, die Gruppe B hydrogenolytisch abspaltet, oder

d) zur Herstellung von Verbindungen der allgemeinen Formel Ib

(siehe Formel Ib)

worin $R^2$, $R^3$, $R^4$, Z, n und m obige Bedeutung besitzen, und $R^{1'}$ niederes Alkyl bedeutet, Verbindungen der allgemeinen Formel VI

(siehe Formel VI)

worin $R^4$ obige Bedeutung besitzt und T für den Rest ZH, worin Z obige Bedeutung besitzt, oder für eine abspaltbare Fluchtgruppe Y, insbesondere Halogen, steht, umsetzt mit Verbindungen der allgemeinen Formel VII

(siehe Formel VII)

worin $R^{1'}$, $R^2$, $R^3$, n und m obige Bedeutung besitzen, und T' für eine abspaltbare Fluchtgruppe Y, insbesondere Halogen, steht, falls T den Rest ZH bedeutet, oder T' für den Rest ZH steht, falls T eine abspaltbare Fluchtgruppe Y bedeutet,

und gewünschtenfalls erhaltene Verbindungen der allgemeinen Formel I, worin $R^1$ Wasserstoff bedeutet, zu entsprechenden Verbindungen der Formel I, worin $R^1$ niederes Alkyl bedeutet, alkyliert, und gewünschtenfalls freie Verbindungen der Formel I in ihre Säureadditionssalze überführt oder die Säureadditionssalze in die freien Verbindungen der Formel I überführt.

Die Umsetzung von Verbindungen der Formel II mit Verbindungen der Formel III gemäß Verfahrensvariante a) kann auf an sich bekannte Weise unter zur Alkylierung von Aminen üblichen Bedingungen erfolgen. Als Fluchtgruppe Y in den Verbindungen der Formel II oder den Verbindungen der Formel III kommen bevorzugt Halogene wie Chlor, Brom oder Jod oder auch organische Sulfonsäurereste in Frage, beispielsweise Reste von Niederalkansulfonsäuren wie z.B. Methansulfonsäure oder von aromatischen Sulfonsäuren wie Benzolsulfonsäure oder durch niederes Alkyl oder Halogen substituierten Benzolsulfonsäuren, z.B. Toluolsulfonsäuren oder Brombenzolsulfonsäuren. Die Umsetzung wird zweckmäßig in Lösung unter basischen Bedingungen durchgeführt. Als Lösungsmittel können inerte organische Lösungsmittel oder auch ein Überschuß der umzusetzenden Aminverbindung dienen. Als inerte organische Lösungsmittel eignen sich niedere Alkohole, z.B. Methanol, oder Dimethylformamid, Acetonitril oder auch Äther, insbesondere cyclische Äther wie Tetrahydrofuran, oder aromatische Kohlenwasserstoffe wie Benzol oder Toluol oder Gemische aus den vorgenannten Lösungsmitteln. Zweckmäßigerweise wird die Reaktion in Gegenwart einer mindestens äquivalenten Menge einer säurebindenden Base durchgeführt. Beispiele geeigneter Basen sind organische Basen wie Alkalimetallcarbonate oder organische Basen, insbesondere tertiäre Amine, z.B. tertiäre Niederalkylamine wie Triäthylamin oder N,N-Dimethylaminopyridin, oder gegebenenfalls auch ein Überschuß der umzusetzenden Aminverbindung.

Die Reaktionstemperatur kann je nach Art der verwendeten Reagenzien variieren und zwischen 0 °C und Siedetemperatur des Lösungsmittels, insbesondere zwischen ca. 20 °C und Siedetemperatur des Lösungsmittels, gewählt werden. Die Reaktionsdauer kann je nach Art der gewählten Reaktionsbedingungen zwischen zwei und zwölf Stunden betragen. Falls $R^1$ Wasserstoff ist, ist es zur Vermeidung von Nebenreaktionen zweckmäßig, einen Überschuß an Aminverbindung einzusetzen. Die erhaltenen Verbindungen der Formel I können von allfälligen geringen Beimengungen an Nebenprodukten leicht z.B. durch eine chromatographische Reinigung befreit werden.

Die Reduktion von Amiden der Formel IV zu Verbindungen der Formel I gemäß Verfahrensvariante b) kann auf an sich bekannte Weise mit zur Reduktion von Amiden geeigneten Reduktionsmitteln erfolgen. Als Reduktionsmittel eignen sich insbesondere komplexe Metallhydride wie Lithiumaluminiumhydrid, Diboran, Natriumborhydrid in Eisessig oder Diisobutylaluminiumhydrid. Die Reduktion erfolgt in einem unter den Reaktionsbedingungen inerten Lösungsmittel, beispielsweise einem Äther, vorzugsweise einem cyclischen Äther wie Tetrahydrofuran, oder Gemischen aus einem cyclischen Äther und einem aromatischen Kohlenwasserstoff wie Toluol. Die Reaktionstemperatur kann zwischen Raumtemperatur und Siedetemperatur des Lösungsmittels liegen. Die Reaktionsdauer kann je nach Art des verwendeten Reduktionsmittels und der gewählten Reaktionsbedingungen zwischen 3 und 10 Stunden betragen.

Die Gewinnung von Verbindungen der Formel Ia aus Verbindungen der Formel V gemäß Verfahrensvariante c) kann auf an sich bekannte Weise durch katalytische Hydrogenolyse erfolgen. Als hydrogenolytisch abspaltbare Gruppen B kommen insbesondere der Benzylrest oder ein im Phenylring substituierter Benzylrest in Frage. Die Hydrogenolyse kann in Gegenwart von zur hydrogenolytischen Debenzylierung geeigneten Katalysatoren bei einem Wasserstoffdruck von 1 bis 10 bar, insbesondere 1 - 6 bar, und Temperaturen von 0 bis 60 °C in einem unter den Reaktionsbedingungen inerten Lösungsmittel durchgeführt werden. Als Katalysatoren eignen sich beispielsweise Palladium auf Kohle, Palladiumhydroxid auf Kohle oder Raney Nickel. Als Lösungsmittel eignen sich beispielsweise niedere Alkohole, z.B. Äthanol, oder Essigsäureethylester, Essigsäure oder aromatische Kohlenwasserstoffe wie Toluol oder deren Gemische, gegebenenfalls auch im Gemisch mit Wasser. Zweckmäßig wird die Hydrogenolyse in angesäuertem Medium, beispielsweise in einem einen Zusatz von Salzsäure enthaltenden Reaktionsmedium, durchgeführt.

Die Umsetzung von Verbindungen der Formel VI mit Verbindungen der Formel VII gemäß Verfahrensvariante d) kann auf an sich bekannte Weise nach zur Äther- oder Thioätherbildung üblichen Methoden beziehungsweise nach zur Aminoalkylierung üblichen Methoden durchgeführt werden.

So werden z.B. Verbindungen der allgemeinen Formel VIc

(siehe Formel VIc)

worin $R^4$ obige Bedeutung besitzt und Z" Sauerstoff oder Schwefel bedeutet, mit Verbindungen der allgemeinen Formel VIIa

(siehe Formel VIIa)

worin $R^{1'}$, $R^2$, $R^3$, n, m und Y obige Bedeutung besitzen, insbesondere Halogeniden der Formel VIIa, umgesetzt, oder Verbindungen der allgemeinen Formel VIb

(siehe Formel VIb)

worin $R^4$ und Y obige Bedeutung besitzen, insbesondere Halogenide der Formel VIb, werden mit Verbindungen der allgemeinen Formel VIIc

(siehe Formel VIIc)

worin $R^{1'}$, $R^2$, $R^3$, Z", n und m obige Bedeutung besitzen umgesetzt. Die Umsetzung erfolgt zweckmäßigerweise in einem unter den Reaktionsbedingungen inerten organischen Lösungsmittel und in Gegenwart einer starken Base, welche fähig ist mit den Alkoholen bzw. Thioalkoholen der Formeln VIc oder VIIc zu den entsprechenden Alkoholaten bzw. Thioalkoholaten zu reagieren. Als starke Basen eignen sich beispielsweise Alkalimetalle, Alkalimetallhydride, Alkalimetallamide oder Alkalimetallhydroxide. Als inerte organische Lösungsmittel eignen sich beispielsweise Äther, insbesondere cyclische Äther wie Tetrahydrofuran oder Dioxan, oder aromatische Kohlenwasserstoffe wie Benzol, Toluol oder Xylol. Gewünschtenfalls kann die Umsetzung auch ohne Zusatz eines Lösungsmittels durchgeführt werden. Die Reaktionstemperatur kann zwischen Raumtemperatur und Siedetemperatur des Reaktionsgemisches liegen und die Reaktionsdauer kann je nach Reaktionsbedingungen zwischen 2 und 10 Stunden betragen.

Sofern T oder T' in den Verbindungen der Formel VI oder den Verbindungen der Formel VII einen $HNR^5$-Rest darstellen, kann die Umsetzung der Verbindungen nach an sich zur Aminoalkylierung üblichen Methoden erfolgen. So kann die Umsetzung beispielsweise unter den für die Umsetzung von Verbindungen der Formel II mit Verbindungen der Formel III angegebenen Bedingungen durchgeführt werden.

Erhaltene Verbindungen der Formel I, worin $R^1$ Wasserstoff bedeutet, können gewünschtenfalls nachträglich in an sich bekannter Weise zu den entsprechenden N-Alkylverbindungen alkyliert werden. Als Alkylierungsmittel kommen Alkylhalogenide, insbesondere Jodide, Alkylsulfate oder Alkylsulfonsäureester in Frage. Zweckmäßigerweise wird die Alkylierung in einem unter den Reaktionsbedingungen inerten organischen Lösungsmittel in Gegenwart einer Base wie beispielsweise einem Alkalimetallcarbonat oder einem tertiären organischen Amin, insbesondere einem tertiären Niederalkylamin durchgeführt. Je nach der verwendeten Base eignen sich als Lösungsmittel Dimethylformamid, Acetonitril, cyclische Äther wie Tetrahydrofuran oder Dioxan, aromatische Kohlenwasserstoffe wie Toluol oder auch niedere Alkohole. Die Umsetzung kann bei Temperaturen zwischen Raumtemperatur und Siedetemperatur des Lösungsmittels erfolgen. Die nachträgliche Alkylierung kann auch als reduktive Alkylierung auf an sich bekannte Weise durch Umsetzen mit einem niederen Aldehyd, insbesondere Formaldehyd, unter reduzierenden Bedingungen erfolgen. Z.B. können die Verbindungen mit dem Aldehyd in Gegenwart eines Reduktionsmittels, beispielsweise Ameisensäure, umgesetzt werden. Sofern in den Verbindungen der Formel I Z für Z' und m für m' stehen, kann die reduktive Alkylierung auch durch Umsetzen der Verbindung mit dem Aldehyd und katalytische Hydrierung des Reaktionsgemisches erfolgen. Als Hydrierungskatalysator eignet sich zum Beispiel Palladium auf Kohle.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Aus Stereoisomerengemischen der Verbindungen der Formel I können die einzelnen stereoisomeren Formen gewünschtenfalls durch übliche Trennverfahren, z.B. fraktionierte Kristallisation geeigneter Salze oder chromatographische Verfahren, angereichert und isoliert werden.

Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäure, oder mit organischen Säuren, beispielsweise niederen aliphatischen Mono- oder Dicarbonsäuren wie Maleinsäure, Fumarsäure, Oxalsäure, Milchsäure, Weinsäure oder Essigsäure, oder Sulfonsäuren, beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure, oder Cyclohexylaminsulfonsäure.

Die Ausgangsverbindungen der Formel III können auf an sich bekannte Weise erhalten werden.

Verbindungen der allgemeinen Formel IIIa

(s. Formel IIIa)

worin $R^4$, Z, n und Y obige Bedeutung besitzen, können beispielsweise erhalten werden, indem man in Verbindungen der allgemeinen Formel X

(s. Formel X)

worin $R^4$, Z und n obige Bedeutung besitzen, die Hydroxygruppe in an sich bekannter Weise in eine Fluchtgruppe Y überführt. So können die Verbindungen der Formel X beispielsweise zur Einführung eines Halogenrestes Y mit Thionylchlorid oder mit Phosphorhalogeniden auf an sich bekannte Weise umgesetzt werden. Sulfonsäurereste Y können auf an sich bekannte Weise eingeführt werden, indem man Verbindungen der Formel X mit einem entsprechenden Sulfonsäurechlorid acyliert.

Verbindungen der Formel X können auf an sich bekannte Weise ausgehend von Verbindungen der Formel VIb hergestellt werden. Zur Herstellung der Verbindungen der Formel X, worin Z Sauerstoff bedeutet, können Verbindungen der Formel VIb, insbesondere Halogenide VIb, beispielsweise mit einem Diol der allgemeinen Formel XI

(s. Formel XI)

worin n obige Bedeutung besitzt, umgesetzt werden, wobei zur Vermeidung von Nebenreaktionen zweckmäßigerweise ein Überschuß des Diols eingesetzt wird. Die Umsetzung kann unter zur Ätherbildung üblichen Bedingungen in Gegenwart einer starken Base wie einem Alkalimetall oder einem Alkalimetallhydrid, -amid oder -hydroxid durchgeführt werden. Zur Herstellung von Verbindungen der Formel X, worin Z für Schwefel steht, können aus den Verbindungen der

Formel VIb auf an sich bekannte Weise in situ erhaltene Alkalimetallsalze von Verbindungen der allgemeinen Formel VIe

(s. Formel VIe)

worin $R^4$ obige Bedeutung besitzt, mit Halogenalkoholen der allgemeinen Formel XII

(s. Formel XII)

worin n obige Bedeutung besitzt und X Halogen bedeutet, umgesetzt werden. Zur Herstellung von Verbindungen der Formel X, worin Z für die Gruppe $NR^5$ steht, können Verbindungen der Formel VIb mit Aminoalkoholen der allgemeinen Formel XIII

(s. Formel XIII)

worin $R^5$ und n obige Bedeutung besitzen, umgesetzt werden. Die Umsetzungen der Verbindungen der Formel VIb zu den Verbindungen der Formel X können unter den vorstehend für die Umsetzung von Verbindungen der Formel VI mit Verbindungen der Formel VII angegebenen Reaktionsbedingungen durchgeführt werden.

Verbindungen der allgemeinen Formel IIIb

(s. Formel IIIb)

worin $R^1$, $R^4$, Z und n obige Bedeutung besitzen, können erhalten werden, indem man Amide der allgemeinen Formel XIV

(s. Formel XIV)

worin $R^1$, $R^4$, Z und n obige Bedeutung besitzen, auf an sich bekannte Weise reduziert. Die Reduktion kann beispielsweise unter den für die Reduktion von Verbindungen der Formel IV vorstehend angegebenen Bedingungen erfolgen.

Verbindungen der Formel IIIb können auch erhalten werden, indem man Verbindungen der Formel IIIa mit einem Amin der allgemeinen Formel IX

(s. Formel IX)

worin $R^1$ obige Bedeutung besitzt,umsetzt. Die Umsetzung kann nach an sich zur Aminoalkylierung üblichen Methoden, beispielsweise bei den vorstehend für die Umsetzung von Verbindungen der Formel II mit Verbindungen der Formel III angegebenen Bedingungen erfolgen. Zur Vermeidung von Nebenreaktionen ist es zweckmäßig, einen mehrfachen Überschuß an Amin der Formel IX einzusetzen.

Verbindungen der allgemeinen Formel IIIc

(s. Formel IIIc)

worin $R^1$, $R^4$, Z' und n obige Bedeutung besitzen, können auch aus Verbindungen der allgemeinen Formel XVII

(s. Formel XVII)

worin $R^1$, $R^4$, Z', n und B obige Bedeutung besitzen, durch hydrogenolytische Abspaltung der Gruppe B erhalten werden. Die Abspaltung kann durch katalytische Hydrierung beispielsweise unter den zur Herstellung von Verbindungen der Formel Ia gemäß Verfahrensvariante c) angegebenen Bedingungen erfolgen. Verbindungen der Formel XVII können ihrerseits durch Umsetzung von entsprechenden Verbindungen der Formel IIIa mit Aminen der allgemeinen Formel IX'

(s. Formel IX')

worin $R^1$ und B obige Bedeutung besitzen, hergestellt werden.

Amide der Formel XIV können erhalten werden, indem man Säuren der allgemeinen Formel XV

(s. Formel XV)

worin $R^4$, Z und n obige Bedeutung besitzen, oder deren reaktionsfähige Säurederivate mit Aminen der Formel IX auf an sich bekannte Weise umsetzt. So können reaktionsfähige Derivate der Säuren XV, insbesondere deren Säurehalogenide, vorzugsweise -chloride, Ester und gemischte Anhydride, z.B. gemischte Anhydride mit Sulfonsäuren, nach an sich zur Bildung von Amid-Gruppierungen durch Aminoacylierung üblichen Methoden mit den Aminen der Formel IX umgesetzt werden. Die Aminoacylierung kann in einem unter den Reaktionsbedingungen inerten Lösungsmittel bei Temperaturen zwischen Raumtemperatur und Siedetemperatur des Lösungsmittels erfolgen. Als Lösungsmittel eignen sich halogenierte Kohlenwasserstoffe wie Dichlormethan oder Chloroform, aromatische Kohlenwasserstoffe wie Benzol, Toluol, Xylol oder Chlorbenzol, cyclische Äther wie Tetrahydrofuran oder Dioxan oder Gemische dieser Lösungsmittel. Die Aminoacylierung kann gegebenenfalls, insbesondere wenn ein Säurehalogenid oder gemischtes Säureanhydrid verwendet wird, in Gegenwart eines säurebindenden Reagenzes durchgeführt werden. Als säurebindende Mittel eignen sich insbesondere organische Basen, beispielsweise tertiäre Niederalkylamine und Pyridine. Falls Säurehalogenide eingesetzt werden, kann die Reaktion auch in wäßrigem Medium und in Gegenwart anorganischer Basen in an sich bekannter Weise nach der Methode von Schotten-Baumann durchgeführt werden. Falls die Säure selbst eingesetzt wird, so wird die Umsetzung zweckmäßigerweise in Gegenwart eines aus der Peptidchemie als geeignet bekannten Kopplungsreagenzes durchgeführt. Als Beispiel von Kopplungsreagenzien, welche die Amidbildung mit der freien Säure dadurch fördern, daß sie mit der Säure in situ unter Bildung eines reaktionsfähigen Säurederivates reagieren, seien insbesondere genannt Alkyl-, vorzugsweise Cycloalkylcarbodiimide wie Dicyclohexylcarbodiimid, oder N-Niederalkyl-2-halogenpyridiniumsalze, insbesondere Halogenide oder Tosylate, vorzugsweise N-Methyl-2-chlorpyridiniumjodid (siehe z.B. Mukayama in angew. Chemie 91, 789 bis 912). Die Umsetzung in Gegenwart eines Kopplungsreagenzes kann beispielsweise bei Temperaturen von -30 bis +100 °C unter Benutzung

von Lösungsmitteln wie halogenierten Kohlenwasserstoffen und/oder aromatischen Lösungsmitteln in Gegenwart eines säurebindenden Amins durchgeführt werden.

Säuren der Formel XV können erhalten werden, indem man Verbindungen der allgemeinen Formel VIa

(siehe Formel VIa)

worin $R^4$ und Z obige Bedeutung besitzen, mit Halogencarbonsäuren der allgemeinen Formel XVI

(siehe Formel XVI)

worin n und X obige Bedeutung besitzen, umsetzt. Die Umsetzung von Verbindungen der Formel VIa mit den Säuren der Formel XVI kann auf an sich bekannte Weise unter zur Äther- oder Thioätherbildung bzw. unter zur Aminoalkylierung üblichen Reaktionsbedingungen erfolgen. So können Salze der Säuren der Formel XVI mit Alkalimetallsalzen solcher Verbindungen der Formel VIa, worin Z für Sauerstoff oder Schwefel steht, oder mit Verbindungen der Formel VIa, worin Z für $NR^5$ steht, unter basischen Bedingungen umgesetzt werden. Die Reaktion kann beispielsweise bei den für die Umsetzung von Verbindungen der Formel VI mit Verbindungen der Formel VII angegebenen Bedingungen durchgeführt werden.

Säuren der Formel XV, worin n für 3 steht, können auch erhalten werden, indem man Verbindungen der Formel VIa mit Acrylsäurederivaten, vorzugsweise Acrylnitril, umsetzt und das Reaktionsprodukt anschließend hydrolysiert. Das Reaktionsprodukt der Verbindungen der Formel VIa mit Acrylnitril kann auch direkt zu solchen Aminen der Formel IIIb, worin n für 3 und $R^1$ für Wasserstoff stehen, reduziert werden.

Verbindungen der allgemeinen Formel II sind bekannt oder können nach an sich bekannten Methoden oder analog zu an sich bekannten Methoden hergestellt werden. Beispielsweise können Verbindungen der Formel II ausgehend von entsprechenden Säuren der allgemeinen Formel VIII

(s. Formel VIII)

worin $R^2$, $R^3$ und m obige Bedeutung besitzen, erhalten werden. So können reaktionsfähige Säurederivate der Säuren der Formel VIII mit Aminen der Formel IX zu entsprechenden Amiden umgesetzt und diese anschließend zu Verbindungen der allgemeinen Formel IIa

(s. Formel IIa)

worin $R^1$, $R^2$, $R^3$ und m obige Bedeutung besitzen, reduziert werden. Säuren der Formel VIII können auch zu den entsprechenden Alkoholen reduziert und in diesen auf an sich bekannte Weise die Hydroxygruppe in die Gruppe Y überführt werden, um Verbindungen der allgemeinen Formel IIb

(s. Formel IIb)

worin $R^2$, $R^3$, m und Y obige Bedeutung besitzen, zu erhalten. Säuren der Formel VIII sind bekannt oder können auf an sich bekannte Weise erhalten werden.

Die Amidverbindungen der allgemeinen Formel IV können durch Umsetzung der Säuren der Formel XV mit Aminen der Formel IIa nach zur Amidbildung üblichen Methoden unter zur Aminoacylierung üblichen Bedingungen erhalten werden.

Verbindungen der Formel V können durch Umsetzung von Verbindungen der allgemeinen Formel IIId

(s. Formel IIId)

worin $R^4$, Z', n und Y obige Bedeutung besitzen, mit Verbindungen der allgemeinen Formel XVIII

(s. Formel XVIII)

worin $R^2$, $R^3$, m' und B obige Bedeutung besitzen, erhalten werden. Die Umsetzung kann in an sich zur Aminoalkylierung bekannter Weise beispielsweise unter den für die Umsetzung von Verbindungen der Formel II mit Verbindungen der Formel III angegebenen Bedingungen erfolgen.

Verbindungen der Formel XVIII können erhalten werden, indem man entsprechende Säuren der Formel VIII mit einem Amin der allgemeinen Formel IX"

(s. Formel IX")

worin B obige Bedeutung besitzt, auf an sich bekannte Weise umsetzt und das erhaltene Amid reduziert.

Verbindungen der Formel XVIII können auch ausgehend von Aminen der allgemeinen Formel IIc

(s. Formel IIc)

worin $R^2$, $R^3$ und m' obige Bedeutung besitzen, erhalten werden. So können Amine der Formel IIc mit einem gegebenenfalls substituierten Benzaldehyd umgesetzt und die als Reaktionsprodukt erhaltene Schiff'sche Base anschließend zu Verbindungen der Formel XVIII reduziert werden. So können die Verbindungen der Formel IIc mit dem Aldehyd unter reduzierenden Bedingungen, z.B. unter den vorstehend für die reduktive Alkylierung von Verbindungen der Formel I zur nachträglichen Einführung eines Alkylrestes $R^1$ angegebenen Bedingungen, umgesetzt werden.

Verbindungen der allgemeinen Formel VId

(s. Formel VId)

worin $R^4$ obige Bedeutung besitzt, sind bekannt. Die anderen Verbindungen der Formel VI können aus Verbindungen der Formel VId auf an sich bekannte Weise hergestellt werden. Zur Herstellung der Verbindungen der Formel VIb kann beispielsweise die Hydroxygruppe der Verbindungen der Formel VId auf an sich bekannte Weise in eine Gruppe Y überführt werden. Verbindungen der Formel VIe können ausgehend von Verbindungen der Formel VId bzw. Verbindungen der Formel VIb erhalten werden. Hierzu wird die Hydroxygruppe der Verbindungen der Formel VId zunächst in

Halogen überführt, und die gebildeten Halogenide der Formel VIb werden anschließend mit Kaliumthioacetat zu Verbindungen der allgemeinen Formel XIX

(s. Formel XIX)

worin $R^4$ obige Bedeutung besitzt, umgesetzt. Aus diesen wird dann durch Behandeln mit einem Alkalimetallalkoxid das Alkalimetallsalz der Verbindung der Formel VIe erhalten, welches direkt für die weiteren Umsetzungen eingesetzt werden kann.

Verbindungen der allgemeinen Formel VIf

(s. Formel VIf)

worin $R^4$ und $R^5$ obige Bedeutung besitzen, können erhalten werden, indem man Verbindungen der Formel VIb mit einem Überschuß eines Amins der allgemeinen Formel XX

(s. Formel XX)

worin $R^5$ obige Bedeutung besitzt, umsetzt, oder die Verbindung der Formel VIb mit einem Amin der allgemeinen Formel XX'

(s. Formel XX')

worin $R^5$ und B obige Bedeutung besitzen, umsetzt und anschließend die Gruppe B hydrogenolytisch abspaltet. Verbindungen der Formel VIf, worin die $NR^5$-Gruppe direkt an ein Ringkohlenstoffatom gebunden ist, können auch durch Umsetzung des dem betreffenden Alkohol VId entsprechenden Ketons mit einem Amin der Formel XX und Reduktion der gebildeten Schiff'schen Base unter zur reduktiven Alkylierung üblichen Bedingungen erfolgen.

Verbindungen der Formel VII können auf an sich bekannte Weise erhalten werden, indem man einen Halogenalkohol der Formel XII mit einem Amin der allgemeinen Formel IId

(s. Formel IId)

worin $R^{1'}$, $R^2$, $R^3$ und m obige Bedeutung besitzen, umsetzt zu Verbindungen der allgemeinen Formel VIIb

(s. Formel VIIb)

worin $R^{1'}$, $R^2$, $R^3$, m und n obige Bedeutung besitzen. Aus den Verbindungen der Formel VIIb können die übrigen Verbindungen der Formel VII auf an sich bekannte Weise durch Austausch der Hydroxygruppe gegen einen anderen Rest T' erhalten werden. So kann die Hydroxygruppe der Verbindungen der Formel VIIb zunächst auf an sich bekannte Weise in eine Gruppe Y umgewandelt werden, und die so erhaltenen Verbindungen der Formel VIIa können auf an sich bekannte Weise weiter umgesetzt werden zu Verbindungen der Formel VII, worin T' eine Gruppe ZH darstellt, worin Z Schwefel oder $NR^5$ bedeutet.

Die nachstehenden Beispiele sollen die Erfindung näher erläutern, ohne jedoch ihren Umfang zu beschränken.

Sofern in den nachstehenden Beispielen Rf-Werte zur Charakterisierung von Substanzen angegeben sind. werden diese bestimmt durch Dünnschichtchromatographie auf Kieselgel/Glasplatten (Hersteller Merck AG, Typ Si60F254). Sofern nichts anderes angegeben wird, wurde als Laufmittel Dichlormethan/Diäthyläther (1:1) verwendet.

Die in den Beispielen angegebenen stereoisomeren Formen der Verbindungen können bis zu ca. 5% an anderen stereoisomeren Formen dieser Verbindungen enthalten.

Beispiel 1:

N-(2-[((1S,2S,5S)-6,6-Dimethylbicyclo[3.1.1]hept-2-yl)-methoxy]-ethyl)-N-(phenethyl)-N-methylamin.

A) 60 g (-)-cis-Myrtanol (= ((1S,2S,5S)-6,6-Dimethylbicyclo[3.1.1]hept-2-yl)-methanol) wurden in 500 ml Toluol gelöst. Zu der Lösung wurden unter Rühren und Außenkühlung 9,9 g metallisches Natrium portionsweise zugegeben. Anschließend wurde das Reaktionsgemisch bis zur völligen Lösung des Natriums bei einer Temperatur von 80 °C gerührt. Sodann wurden 89 g Dinatriumjodidacetat zugesetzt und das Reaktionsgemisch 40 Stunden unter Rückfluß gekocht. Zur Aufarbeitung wurde das Reaktionsgemisch nach Abkühlen mit Dichlormethan verdünnt und zweimal mit wäßriger 2N Natriumhydroxidlösung extrahiert. Die wäßrige Phase wurde abgetrennt, mit 6N Salzsäurelösung sauer gestellt und dreimal mit Dichlormethan extrahiert. Die vereinigten Dichlormethanextrakte wurden über Natriumsulfat getrocknet, und das Lösungsmittel wurde unter vermindertem Druck abdestilliert. Als Rückstand wurden 13,4 g flüssige cis-Myrtanyloxyessigsäure erhalten, welche ohne Reinigung in der nächsten Stufe weiterverarbeitet wurde.

B) 7,7 g des vorstehend erhaltenen Produktes wurden in 50 ml Dichlormethan gelöst. Zu der Lösung wurden bei einer Temperatur von 0 °C 5 ml Methansulfonsäurechlorid zugegeben. Dann wurden 11 ml Triethylamin und eine geringe Menge Pyridin vorsichtig bei einer Temperatur von 0 °C zugetropft und das Reaktionsgemisch wurde anschließend noch 2 Stunden bei 0 °C und weitere 2 Stunden bei Raumtemperatur gerührt. Zu der das gemischte Anhydrid aus der cis-Myrtanyloxyessigsäure und Methansulfonsäure enthaltenden Lösung wurden 5 ml N-Methyl-N-phenethylamin gegeben und das Reaktionsgemisch wurde weitere 5 Stunden bei Raumtemperatur gerührt. Zur Aufarbeitung wurde das Reaktionsgemisch zweimal mit je 100 ml wäßriger 2N Natriumhydroxidlösung extrahiert. Die organische Phase wurde über Natriumsulfat getrocknet und unter vermindertem Druck eingeengt. Das als

Rückstand verbliebene ölige Rohprodukt wurde mittels Chromatographie an Kieselgel unter leicht erhöhtem Druck (Flash-Chromatographie) unter Verwendung von Ether/Dichlormethan als Elutionsmittel gereinigt. Es wurden 6,8 g öliges N-(Methyl)-N-(phenethyl)-[((1S,2S,5S)-6,6-dimethylbicyclo[3.1.1]hept-2-yl)-methoxy]-acetamid erhalten. Rf-Wert = 0,4.

C) 1,2 g Lithiumaluminiumhydrid wurden unter Stickstoffbegasung in 20 ml eines Gemisches Tetrahydrofuran/Toluol (7 : 3) aufgeschlämmt. Zu der Aufschlämmung wurden in der Siedehitze eine Lösung von 6,8 g des vorstehend unter B) erhaltenen öligen Produktes in 30 ml des Tetrahydrofuran/Toluol-Gemisches getropft, und das Reaktionsgemisch wurde 5 Stunden am Rückfluß gekocht. Zur Aufarbeitung wurden dem Reaktionsgemisch nach Erkalten nacheinander 1,2 ml Wasser, 1,2 ml 15%-ige wäßrige Natriumhydroxidlösung und nochmals 3,5 ml Wasser vorsichtig zugegeben, um überschüssiges Lithiumaluminiumhydrid zu zersetzen. Nach Beendigung der Reaktion wurde das Reaktionsgemisch filtriert und das Filtrat unter vermindertem Druck eingedampft. Der verbleibende Rückstand wurde in Ether gelöst und zweimal mit je 50 ml 2N wäßriger Salzsäurelösung extrahiert. Die salzsaure wäßrige Phase wurde abgetrennt, alkalisch gestellt und mit Ether extrahiert. Der Etherextrakt wurde über Natriumsulfat getrocknet und unter vermindertem Druck eingeengt. Die verbleibende ölige rohe Titelverbindung wurde durch Chromatographie an Kieselgel unter Verwendung von Ether/Dichlormethan (1 : 1) als Elutionsmittel gereinigt. Es wurden 2,5 g N-(2-[((1S,2S,5S)-6,6-Dimethylbicyclo[3.1.1]hept-2-yl)-methoxy]-ethyl)-N-(phenethyl)-N-methylamin erhalten. Rf-Wert = 0,15.

Zur Überführung in ihr Hydrochlorid wurde die Verbindung in Ether gelöst, und die Lösung wurde mit gasförmigem Chlorwasserstoff versetzt und anschließend eingeengt. Hierbei wurde das amorphe Hydrochlorid des N-(2-[((1S,2S,5S)-6,6-Dimethylbicyclo[3.1.1]hept-2-yl)-methoxy]-ethyl)-N-(phenethyl)-N-methylamin mit einem Schmelzpunkt von 160 °C erhalten.
$[\alpha]_D^{20}$ = -5,1° (c = 1; in Methanol)

Beispiel 2:

N-(3-[((1S,2S,5S)-6,6-Dimethylbicyclo[3.1.1]hept-2-yl)-methoxy]-propyl)-N-(phenethyl)-N-methylamin.

A) 51 g cis-Myrtanol wurden in wenig Dichlormethan gelöst. Zu der Lösung wurden 5,5 g Benzyl-trimethylammoniumhydroxid (= Triton B) gegeben. Dann wurden dem Reaktionsgemisch unter Eiskühlung 32,5 ml Acrylnitril zugetropft, wobei die Innentemperatur zwischen 30 und 40 °C reguliert wurde. Nach beendeter Reaktion wurde das Reaktionsgemisch mit 200 ml Dichlormethan verdünnt und zweimal mit je 100 ml wäßriger verdünnter Salzsäurelösung gewaschen. Anschließend wurde die organische Phase eingedampft. Es wurden 71 g rohes 3-[((1S,2S,5S)-6,6-Dimethylbicyclo[3.1.1]hept-2-yl)-methoxy]-propionsäurenitril als dünnflüssiges Öl erhalten. Rf-Wert = 0,6.

B) 60 g des vorstehend erhaltenen öligen Produktes wurden in 200 ml konzentrierter wäßriger Salzsäurelösung 8 Stunden bei Raumtemperatur gerührt. Dann wurde das Reaktionsgemisch mit Dichlormethan extrahiert. Der Dichlormethanextrakt wurde eingeengt, und der verbleibende Rückstand wurde durch Chromatographie an Kieselgel unter Verwendung von Dichlormethan/Ether (1 : 1) als Elutionsmittel gereinigt. Es wurden 28 g 3-[((1S,2S,5S)-6,6-Dimethylbicyclo[3.1.1]hept-2-yl)-methoxy]-propionsäure erhalten. Rf-Wert = 0,15.

C) 8,1 g der vorstehend erhaltenen Säure wurden wie in Beispiel 1B) beschrieben in Dichlormethan gelöst und durch Umsetzung mit 5 ml Methansulfonsäurechlorid in das gemischte Anhydrid überführt, welches mit 10 ml N-Methyl-N-phenethylamin, 11,1 ml Triethylamin und einer geringen Menge Pyridin in Dichlormethan weiter wie in Beispiel 1B) beschrieben umgesetzt wurde. Das erhaltene Rohprodukt wurde durch Chromatographie an Kieselgel unter Verwendung von Dichlormethan/Ether (1 : 1) als Elutionsmittel gereinigt. Es wurden 9 g N-Methyl-N-(phenethyl)-3-[((1S,2S,5S)-6,6-dimethylbicyclo[3.1.1]hept-2-yl)-methoxy]-propionamid erhalten. Rf-Wert = 0,5.

D) 8 g des vorstehend erhaltenen Amides wurden wie in Beispiel 1 C) beschrieben mit 1,4 g Lithiumaluminiumhydrid in 50 ml eines Gemisches aus Tetrahydrofuran/Toluol (7 : 3) reduziert. Das Reaktionsgemisch wurde wie in Beispiel 1 C) beschrieben aufgearbeitet. Es wurden 3 g N-(3-[((1S,2S,5S)-6,6-Dimethylbicyclo[3.1.1]hept-2-yl)-methoxy]-propyl)-N-(phenethyl)-N-methylamin erhalten. Rf-Wert = 0,15.

Beispiel 3:

N-(4-[2-((1S,2S,5S)-6,6-Dimethylbicyclo[3.1.1]hept-2-yl)-ethoxy]-butyl)-N-(phenethyl)-N-methylamin.

A) 200 g cis-Dihydronopol (= 2-((1S,2S,5S)-6,6-Dimethylbicyclo[3.1.1]hept-2-yl)-ethanol) wurden in 340 ml Toluol gelöst. Zu der Lösung wurden tropfenweise 200 ml Thionylchlorid zugesetzt. Dann wurden 5 ml Dimethylformamid zugesetzt und das Reaktionsgemisch wurde 4 Stunden unter Rückfluß gekocht. Zur Aufarbeitung wurde das Lösungsmittel unter vermindertem Druck abdestilliert, der Rückstand wurde in 300 ml Toluol aufgenommen, wiederum zur Trockene eingeengt und nochmals in 300 ml Toluol aufgenommen und zur Trockene eingedampft. Es wurden 206 g rohes 2-((1S,2S,5S)-6,6-Dimethylbicyclo[3.1.1]hept-2-yl)-ethylchlorid erhalten, welche ohne weitere Reinigung in der nächsten Stufe weiterverarbeitet wurden.

B) 12,2 g elementares Natrium wurden portionsweise über 12 Stunden verteilt bei einer Temperatur von 90 °C in 320 ml 1,4-Butandiol gelöst. Zu dieser Lösung wurden dann tropfenweise 80 g 2-((1S,2S,5S)-6,6-Dimethylbicyclo[3.1.1]hept-2-yl)-ethylchlorid zugegeben und das Reaktionsgemisch 15 Stunden auf 130 °C erhitzt. Zur Aufarbeitung wurde das Reaktionsgemisch auf Raumtemperatur abgekühlt, dann wurden 300 ml einer 5 %-igen wäßrigen Salzsäurelösung hinzugegeben und mit Ether extrahiert. Nach Einengen des Etherextraktes wurden 95,2 g rohes 4-[2-((1S,2S,5S)-6,6-Dimethylbicyclo[3.1.1]hept-2-yl)-ethoxy]-butanol erhalten.

C) 90 g des vorstehend erhaltenen Produktes wurden in 110 ml Toluol gelöst. Zu der Lösung wurden 1 ml Dimethylformamid gegeben und anschließend bei Raumtemperatur tropfenweise 62 ml Thionylchlorid zugesetzt, und das Reaktionsgemisch wurde anschließend 3 Stunden am Rückfluß gekocht. Das Reaktionsgemisch wurde wie in Beispiel 3 A) beschrieben aufgearbeitet. Es wurden 95,1 g rohes 4-[2-((1S,2S,5S)-6,6-Dimethylbicyclo[3.1.1]hept-2-yl)-ethoxy]-butylchlorid erhalten, welches sofort in der nachfolgenden Reaktionsstufe weiterverarbeitet wurde. Rf-Wert = 0,7

D) 5 g des vorstehend erhaltenen Produktes wurden mit 3,2 ml N-Methyl-N-phenethylamin und 2,9 ml Triethylamin 5 Stunden bei einer Badtemperatur von 130 °C unter Rückfluß gerührt. Nach dem Erkalten wurden dem Reaktionsgemisch 100 ml Dichlormethan zugefügt, und die erhaltene Lösung wurde mit 50 ml 2N wäßriger Natriumhydroxidlösung und mit 50 ml Wasser gewaschen, über Natriumsulfat getrocknet und eingedampft. Die als Rückstand verbleibende rohe Titelverbindung wurde durch Chromatographie an Kieselgel unter Verwendung von Dichlormethan/Ether (1 : 1) als Elutionsmittel gereinigt. Es wurden 4,4 g N-(4-[2-((1S,2S,5S)-6,6-Dimethylbicyclo[3.1.1]hept-2-yl)-ethoxy]-butyl)-N-(phenethyl)-N-methylamin erhalten. Rf-Wert = 0,15.

Zur Überführung in ihr Hydrochlorid wurde die Substanz in Ether gelöst, die Lösung mit gasförmigem Chlorwasserstoff versetzt und anschließend eingeengt. Es wurden 4,4 g amorphes Hydrochlorid des N-(4-[2-((1S,2S,5S)-6,6-Dimethylbicyclo[3.1.1]hept-2-yl)-ethoxy]-butyl)-N-(phenethyl)-N-methylamin mit einem Schmelzpunkt von 60 °C erhalten.
$[\alpha]_D^{20}$ = -11,5° (c = 1; Methanol)

Beispiel 4:

N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-methylamin.

7 g 4-[2-((1S,2S,5S)-6,6-Dimethylbicyclo[3.1.1]hept-2-yl)-ethoxy]-butylchlorid, 5,8 g N-Methyl-homoveratrylamin, 4 ml Triethylamin und eine katalytische Menge Kaliumjodid wurden in 10 ml Acetonitril 8 Stunden am Rückfluß gekocht. Dann wurde die Reaktion abgebrochen und das Reaktionsgemisch nach Erkalten mit 150 ml Dichlormethan versetzt. Die gebildete Lösung wurde mit 70 ml wäßriger 2N Natriumhydroxidlösung und anschließend zweimal mit Wasser gewaschen, über Natriumsulfat getrocknet, und das Lösungsmittel wurde abdestilliert. Die als Rückstand verbliebene rohe Titelverbindung wurde durch Chromatographie an Kieselgel unter Verwendung von Essigsäureethylester/Methanol (8 : 2) als Elutionsmittel gereinigt. Es wurden 4,8 g N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-methylamin erhalten. Rf-Wert = 0,2. Daneben konnten 2,3 g nicht umgesetztes 4-[2-((1S,2S,5S)-6,6-Dimethylbicyclo[3.1.1]hept-2-yl)-ethoxy]-butylchlorid isoliert werden. Rf-Wert = 0,6.

Die Titelbase wurde wie in Beispiel 3D beschrieben in ihr Hydrochlorid überführt. Es wurden 4,8 g N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-methylamin-Hydrochlorid erhalten.
$[\alpha]_D^{20}$ = -9,5° (c = 1; Methanol)

Beispiel 5:

N-(3-[2-((1S,2S,5S)-6,6-Dimethylbicyclo[3.1.1]hept-2-yl)-ethoxy]-propyl)-N-(phenethyl)-N-methylamin.

A) 15 g 3-[2-((1S,2S,5S)-6,6-Dimethylbicyclo[3.1.1]hept-2-yl)-ethoxy]-propanol (hergestellt analog Beispiel 3 B) aus 2-((1S,2S,5S)-6,6-Dimethylbicyclo[3.1.1]hept-2-yl)-ethylchlorid und 1,3-Propandiol) wurden in 50 ml Dichlormethan gelöst. Zu der Lösung wurden bei einer Temperatur von 0 °C nacheinander 9,2 ml Methansulfonsäurechlorid, 20,4 ml Triethylamin und eine geringe Menge Pyridin zugegeben. Das Reaktionsgemisch wurde 2 Stunden bei Raumtemperatur gerührt. Dann wurde das Lösungsmittel unter vermindertem Druck abdestilliert. Das als Rückstand verbleibende 3-[2-((1S,2S,5S)-6,6-Dimethylbicyclo[3.1.1]hept-2-yl)-ethoxy]-propylmesylat wurde sofort in der nachfolgenden Reaktionsstufe weiterverarbeitet.

B) Das vorstehend erhaltene Produkt wurde in 20 ml N-Methyl-N-phenethylamin gelöst. Die Lösung wurde mit einer katalytischen Menge Kupfer-I-jodid und etwas Natriumcarbonat versetzt, und das Reaktionsgemisch wurde 6 Stunden bei einer Badtemperatur von 100 °C gerührt. Nach dem Erkalten wurde zur Aufarbeitung mit 150 ml Dichlormethan verdünnt und die organische Phase mit gesättigter wäßriger Hydrogencarbonatlösung und anschließend mit Wasser gewaschen, über Natriumsulfat getrocknet und eingedampft. Das verbleibende Rohprodukt wurde durch Chromatographie an Kieselgel unter Verwendung von Dichlormethan/Ether (1 : 1) von Verunreinigungen befreit und dann unter Zusatz von Methanol von der Kieselgelsäure eluiert. Es wurden 7,8 g N-(3-[2-((1S,2S,5S)-6,6-Dimethylbicyclo[3.1.1]hept-2-yl)-ethoxy]-propyl)-N-(phenethyl)-N-methylamin erhalten.
Rf-Wert = 0,1 (Laufmittel Dichlormethan/Ether 1 : 1).

C) 25,5 g der vorstehend erhaltenen Titelbase wurden in 30 ml Tetrahydrofuran gelöst. Zu der Lösung wurden 4,3 ml Ortho-Phosphorsäure gegeben. Das Reaktionsgemisch wurde 30 Minuten bei Raumtemperatur stehengelassen. Sodann wurde das Lösungsmittel unter vermindertem Druck abgedampft und der erhaltene Rückstand aus 30 ml Isopropanol umkristallisiert. Das so erhaltene rohe Hydrogenphosphat der Titelverbindung wurde nochmals in 100 ml Ether bei Rückflußtemperatur gerührt, dann wurde das Lösungsmittel über eine Glasfritte abgesaugt und das verbleibende Kristallisat am Ölpumpenvakuum getrocknet. Es wurden 29,2 g kristallines N-(3-[2-((1S,2S,5S)-6,6-Dimethylbicyclo[3.1.1]hept-2-yl)-ethoxy]-propyl)-N-(phenethyl)-N-methylammonium-dihydrogenphosphat mit einem Schmelzpunkt von 103 °C erhalten.

$[\alpha]_D^{20}$ = -10,4 (c = 1; Methanol)

Beispiel 6:

N-(3-[2-((1S,2S,5S)-6,6-Dimethylbicyclo[3.1.1]hept-2-yl)-ethylthio]-propyl)-N-(phenethyl)-N-methylamin.

A) 15,6 g Triphenylphosphin wurden in 300 ml Acetonitril gelöst und zu der Lösung wurden 3,02 ml Brom tropfenweise unter starkem Rühren bei einer Temperatur von 0 °C zugegeben. Dann wurde eine Lösung von 10 g cis-Dihydronopol (= 2-((1S,2S,5S)-6,6-Dimethylbicyclo[3.1.1]hept-2-yl)-ethanol) in 100 ml Acetonitril zugegeben und das Reaktionsgemisch 5 Stunden auf 100 °C (Badtemperatur) erhitzt. Zur Aufarbeitung wurde das Lösungsmittel unter vermindertem Druck abdestilliert und der Rückstand über Kieselgel unter Verwendung von Dichlormethan als Elutionsmittel chromatographisch gereinigt. Es wurden 12,0 g 2-((1S,2S,5S)-6,6-Dimethylbicyclo[3.1.1]hept-2-yl)-ethylbromid erhalten.

B) 4 g des vorstehend erhaltenen Produktes wurden in 20 ml Dimethylformamid gelöst, zu der Lösung wurden 2,0 g Kaliumthioacetat zugegeben und das Reaktionsgemisch 14 Stunden bei Raumtemperatur gerührt. Anschließend wurde das ausgefallene Kaliumbromid abfiltriert und das Lösungsmittel wurde unter vermindertem Druck abdestilliert. Der verbleibende Rückstand wurde chromatographisch über Kieselgel unter Verwendung von Toluol/Cyclohexan als Elutionsmittel gereinigt. Es wurden 3,8 g 2-((1S,2S,5S)-6,6-Dimethylbicyclo[3.1.1]hept-2-yl)-ethylthioacetat erhalten. Rf-Wert = 0,4 (Laufmittel Toluol/Cyclohexan 1 : 1).

C) 3,8 g des vorstehend erhaltenen Produktes wurden in wenig absolutem Methanol gelöst. Zu der Lösung wurden unter Feuchtigkeitsausschluß 1,05 g Natriummethylat gegeben, und die Lösung wurde 3 Stunden bei Raumtemperatur gerührt. Sodann wurden zu der das Natriumsalz des 2-((1S,2S,5S)-6,6-Dimethylbicyclo[3.1.1]hept-2-yl)-thioethanol enthaltenden Lösung 1,6 ml 3-Brompropanol zugesetzt und das Reaktionsgemisch 8 Stunden am Rückfluß gekocht. Während dieser Zeit wurden noch zweimal je 0,2 ml 3-Brompropanol nachgefügt. Zur Aufarbeitung wurde das Reaktionsgemisch auf wäßrige 6N Salzsäure gegossen und die Mischung wurde zweimal mit je 100 ml Dichlormethan extrahiert. Die organischen Extrakte wurden über Natriumsulfat getrocknet und eingedampft. Der verbleibende Rückstand wurde chromatographisch über Kieselgel unter Verwendung von Essigsäureethylester/ n-Hexan (1 : 4) als Elutionsmittel gereinigt. Es wurden 1,8 g 3-[2-((1S,2S,5S)-6,6-Dimethylbicyclo[3.1.1]hept-2-yl)-ethylthio]-propanol erhalten. Rf-Wert = 0,4.

D) 1,8 g des vorstehend erhaltenen Produktes wurden in 30 ml Dichlormethan gelöst. Zu der Lösung wurden bei einer Temperatur von 0 °C 0,65 ml Methansulfonsäurechlorid gegeben, dann wurden vorsichtig 1,5 ml Triethylamin zugefügt. Das Reaktionsgemisch wurde 2 Stunden bei Raumtemperatur gerührt. Zur Aufarbeitung wurde dann das Lösungsmittel unter vermindertem Druck abdestilliert. Der das gebildete 3-[2-((1S,2S,5S)-6,6-Dimethylbicyclo[3.1.1]hept-2-yl)-ethylthio]-propyl)-methansulfonat enthaltende Rückstand wurde in 30 ml Acetonitril gelöst. Zu der Lösung wurden 0,9 g N,N-Dimethylaminopyridin und 1,2 ml N-Methyl-N-phenethylamin gegeben, und das Reaktionsgemisch wurde 16 Stunden unter Rückfluß gekocht. Zur Aufarbeitung wurde das Acetonitril unter vermindertem Druck abdestilliert, und der verbleibende Rückstand wurde chromatographisch über Kieselgel unter Verwendung von Dichlormethan/Methanol (9 : 1) als Elutionsmittel gereinigt. Es wurden 1,1 g N-(3-[2-((1S,2S,5S)-6,6-Dimethylbicyclo[3.1.1]hept-2-yl)-ethylthio]-propyl)-N-(phenethyl)-N-methylamin erhalten. Rf-Wert = 0,5 (Laufmittel Methanol/Essigsäureethylester 3 : 7).
$[\alpha]_D^{20}$ = -17,0 (c = 1; Methanol)

Beispiel 7:

N,N'-Dimethyl-N-[2-((1S,2S,5S)-6,6-dimethylbicyclo[3.1.1]hept-2-yl)-ethyl]-N'-(phenethyl)-1,2-diaminoethan.

A) Zu 12,4 g 2-((1S,2S,5S)-6,6-Dimethylbicyclo[3.1.1]hept-2-yl)-ethylbromid wurden 3,4 ml N-Methylaminoethanol und 7,3 ml Triethylamin gegeben, und das Reaktionsgemisch wurde 6 Stunden bei Raumtemperatur gerührt. Zur Aufarbeitung wurde das Reaktionsgemisch über Kieselgel chromatographiert unter Verwendung von Dichlormethan/Methanol (8 : 2) als Laufmittel. Es wurden 3,8 g N-Methyl-N-[2-((1S,2S,5S)-6,6-dimethylbicyclo[3.1.1]hept-2-yl)-ethyl]-2-aminoethanol erhalten. Rf-Wert = 0,4. Außerdem wurden 7 g unumgesetztes Ausgangsprodukt zurückgewonnen.

B) 3,8 g des vorstehend erhaltenen Produktes wurden in 50 ml Dichlormethan gelöst. Der Lösung wurden bei einer Temperatur von 0 °C 1,7 ml Methansulfonsäurechlorid und anschließend vorsichtig 3,8 ml Triethylamin zugegeben. Das Reaktionsgemisch wurde 2 Stunden bei Raumtemperatur gerührt. Dann wurde das Lösungsmittel unter vermindertem Druck abdestilliert. Der das gebildete N-Methyl-N-[2-((1S,2S,5S)-6,6-dimethylbicyclo[3.1.1]hept-2-yl)-ethyl]-2-aminoethylmethansulfonat enthaltende Rückstand wurde in 50 ml Acetonitril gelöst. Der Lösung wurden 2,5 ml Triethylamin und 2,8 ml N-Methyl-N-phenethylamin zugesetzt und das Reaktionsgemisch wurde 8 Stunden unter Rückfluß gekocht. Zur Aufarbeitung wurde das Reaktionsgemisch auf 150 ml gesättigte wäßrige Natriumhydrogencarbonatlösung gegossen, und das Gemisch wurde dreimal mit je 50 ml Dichlormethan extrahiert. Die vereinigten Dichlormethanextrakte wurden mit 50 ml Wasser gewaschen und über Natriumsulfat getrocknet. Sodann wurde das Lösungsmittel abgedampft und die als Rückstand verbleibende rohe Titelverbindung wurde durch Chromatographie an Kieselgel unter Verwendung von Dichlormethan/Methanol (9 : 1) als Elutionsmittel gereinigt. Es wurden 2 g N,N'-Dimethyl-N-[2-((1S,2S,5S)-6,6-dimethyl-bicyclo[3.1.1]hept-2-yl)-ethyl]-N'-(phenethyl)-1,2-diaminoethan erhalten. Rf-Wert = 0,5 (Laufmittel: Dichlormethan/Methanol 9 : 1).

Zur Überführung in ihr Hydrochlorid wurde diese Base in Ether gelöst und die Lösung mit gasförmigem Chlorwasserstoff versetzt und eingeengt. Es wurden 1,7 g amorphes Dihydrochlorid der Titelverbindung erhalten.
$[\alpha]_D^{20}$ = -13,2° (c = 1; Methanol)

Beispiel 8:

N-(4-[2-((1S,2S,5S)-6,6-Dimethylbicyclo[3.1.1]hept-2-yl)-ethoxy]-butyl)-N-[2-(3,4-dimethoxyphenyl)-ethyl]-amin.

A) 8,4 g Benzaldehyd und 9 g Homoveratrylamin wurden in 100 ml Ethanol gelöst. Zu der Lösung wurde Raney-Nickel gegeben, und die Mischung wurde 2 Stunden bei Raumtemperatur und einem Wasserstoffdruck von 5 bar hydriert. Anschließend wurde der Katalysator abfiltriert, das Filtrat unter vermindertem Druck eingeengt und der Rückstand in 100 ml Dichlormethan aufgenommen. Die Dichlormethanphase wurde mit Wasser gewaschen, über Natriumsulfat getrocknet und eingedampft. Nach Verreiben des öligen Rückstandes mit Äther wurden 10,9 g festes N-Benzyl-homoveratrylamin erhalten, welches ohne weitere Reinigung weiterverarbeitet wurde.

B) 10,9 g des vorstehend erhaltenen Produktes und 8,5 g 4-[2-((1S,2S,5S)-6,6-Dimethylbicyclo[3.1.1]hept-2-yl)-ethoxy]-butylchlorid wurden in 100 ml Acetonitril gelöst. Nach Zugabe von Kaliumcarbonat wurde das Reaktionsgemisch 4 Stunden am Rückfluß gekocht. Anschließend wurde das Reaktionsgemisch filtriert, das Filtrat eingedampft, und der verbleibende Rückstand wurde durch Chromatographie an Kieselgel unter Verwendung von Dichlormethan/Ether als Elutionsmittel gereinigt. Es wurden 8 g öliges N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-benzylamin erhalten.

C) 3 g des vorstehend erhaltenen Produktes wurden in einem Gemisch aus 100 ml Äthanol und 30 ml Wasser gelöst. Zu der Lösung wurden nacheinander 1,5 g konzentrierte Salzsäurelösung und 2,5 g Hydrierkatalysator (Palladium/Kohle 5 %-ig) gegeben und die Mischung bei Raumtemperatur und einem Wasserstoffdruck von 4 bar hydriert. Nach ca. 3 Stunden war die Wasserstoffaufnahme beendet. Es wurde vom Katalysator abfiltriert und das Filtrat unter vermindertem Druck eingedampft. Der verbleibende Rückstand wurde in 100 ml Wasser aufgenommen und mit Essigsäureäthylester extrahiert. Die organische Phase wurde über Natriumsulfat getrocknet, filtriert und unter vermindertem Druck eingeengt. Die erhaltene rohe Titelverbindung wurde durch Chromatographie über Kieselgel unter Verwendung von Essigsäureethylester/Methanol (7:3) als Elutionsmittel gereinigt. Es wurden 1,8 g öliges N-(4-[2-((1S,2S,5S)-6,6-Dimethylbicyclo[3.1.1]hept-2-yl)-ethoxy]-butyl)-N-[2-(3,4-dimethoxyphenyl)-ethyl]-amin erhalten. Rf-Wert = 0,1 (Laufmittel: Essigsäureethylester/Methanol 9 : 1).

Zur Überführung in ihr Hydrochlorid wurde die Titelbase in Ether gelöst, die Lösung mit gasförmigem Chlorwasserstoff versetzt und anschließend eingeengt. Es wurde das amorphe Hydrochlorid des N-(4-[2-((1S,2S,5S)-6,6-Dimethylbicyclo[3.1.1]hept-2-yl)-ethoxy]-butyl)-N-[2-(3,4-dimethoxyphenyl)-ethyl]-amin erhalten, Schmelzbereich 115 - 125 °C.
$[\alpha]_D^{20}$ = -10,8° (c = 1; in Methanol)

Beispiel 9:

N-[2-((1S,3S,4R)-1-Methyl-4-isopropylcyclohex-3-yloxy)-ethyl]-N-phenethylamin.

A) 2,8 g D-Menthyloxyessigsäure (= ((1S,3S,4R)-1-Methyl-4-isopropylcyclohex-3-yloxy)-essigsäure) wurden in 50 ml Dichlormethan gelöst. Zu der Lösung wurden bei einer Temperatur von 0 °C nacheinander 1,8 ml Methansulfonsäurechlorid, 4 ml Triethylamin und eine geringe Menge Pyridin zugegeben. Das Reaktionsgemisch wurde 2 Std. bei Raumtemperatur gerührt. Dann wurde das Lösungsmittel unter vermindertem Druck abdestilliert. Das als Rückstand verbleibende gemischte Anhydrid aus der D-Menthyloxyessigsäure und Methansulfonsäure wurde sofort in der nachfolgenden Reaktionsstufe weiterverarbeitet.

B) Das vorstehend erhaltene Produkt wurde in 5 ml Phenethylamin gelöst, und das Reaktionsgemisch wurde 4 Stunden bei Raumtemperatur gerührt. Zur Aufarbeitung wurde das Reaktionsgemisch in 100 ml Dichlormethan gelöst, die Lösung zweimal mit je 50 ml 1N wäßriger Salzsäurelösung und einmal mit 50 ml Wasser gewaschen und über Natriumsulfat getrocknet. Anschließend wurde das Lösungsmittel abdestilliert. Als Rückstand verblieben 4 g N-(Phenethyl)-((1S,3S,4R)-1-methyl-4-isopropylcyclohex-3-yloxy)-acetamid. Rf-Wert = 0,6 (Laufmittel: Essigsäureethylester/Methanol 7 : 3).

C) 4 g des vorstehend erhaltenen Amides wurden nach der in Beispiel 1 C beschriebenen Methode in 60 ml Tetrahydrofuran/Toluol-Gemisch mit 0,7 g Lithiumaluminiumhydrid reduziert. Das Reaktionsgemisch wurde anschließend wie in Beispiel 1 C beschrieben aufgearbeitet. Die als Rückstand verbleibende rohe Titelverbindung wurde durch Chromatographie an Kieselgel unter Verwendung von Essigsäureethylester/Methanol (7 : 1) gereinigt. Es wurden 1,9 g öliges N-[2-((1S,3S,4R)-1-Methyl-4-isopropylcyclohex-3-yloxy)-ethyl]-N-phenethylamin erhalten. Rf-Wert = 0,3 (Laufmittel: Essigsäureethylester/Methanol).

Die Titelbase wurde wie in Beispiel 1 C beschrieben in ihr Hydrochlorid überführt. Es wurden 1,7 g N-[2-((1S,3S,4R)-1-Methyl-4-isopropylcyclohex-3-yloxy)-ethyl]-N-phenethylamin-hydrochlorid mit einem Schmelzbereich von 120-145 °C erhalten.
$[\alpha]_D^{20}$ = +59,5° (c = 1; in Methanol)

Beispiel 10:

N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-methylamin.

3 g N-(4-[2-((1S,2S,5S)-6,6-Dimethylbicyclo[3.1.1]hept-2-yl)-ethoxy]-butyl)-N-[2-(3,4-dimethoxyphenyl)-ethyl]-amin (Herstellung siehe Beispiel 8), 1 g Methyljodid und 0,5 g Kaliumcarbonat wurden in 10 ml Acetonitril 2 Stunden bei Raumtemperatur gerührt. Anschließend wurde das Reaktionsgemisch weitere 4 Stunden auf 50 °C erhitzt. Zur Aufarbeitung wurde das Reaktionsgemisch nach Erkalten mit 150 ml Dichlormethan verdünnt, zweimal mit Wasser gewaschen und über Natriumsulfat getrocknet und filtriert. Das Lösungsmittel wurde abdestilliert, und der verbleibende Rückstand wurde durch Chromatographie an Kieselgel unter Verwendung von Essigsäureethylester/Methanol (8 : 2) als Elutionsmittel gereinigt. Es wurden 2,1 g N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-methylamin erhalten. Rf-Wert = 0,1 (Laufmittel: Dichlormethan/Ether 1 : 1).

2,1 g der Titelbase wurden wie in Beispiel 4 beschrieben in ihr Hydrochlorid überführt. Es wurden 2,2 g N-(4-[2-((1S,2S,5S)-6,6-Dimethylbicyclo[3.1.1]hept-2-yl)-ethoxy]-butyl)-N-[2-(3,4-dimethoxyphenyl)-ethyl]-N-methylamin-hydrochlorid erhalten.

$[\alpha]_D^{20}$ = - 9,5° (c = 1; Methanol)

Analog zu den in den vorstehenden Beispielen beschriebenen Methoden wurden auch die in der folgenden Tabelle I angegebenen Verbindungen der Formel I erhalten.

Tabelle I

| Bsp. Nr. | $R^1$ | n | m | Z | $R^2$ | $R^3$ | $R^4$ | Salzform | phys. Konst. Fp., (Schmelzbereich) in °C | phys. Konst. $[\alpha]_D^{20}$ in ° (c=1, $CH_3OH$) |
|---|---|---|---|---|---|---|---|---|---|---|
| 11 | $CH_3$ | 2 | 2 | O | H | H | trans-myr. | HCl | 150 | -15,1 |
| 12 | $CH_3$ | 2 | 2 | O | H | H | L-menth. | HCl | 80-100 | -53,5 |
| 13 | H | 2 | 2 | O | H | H | L-menth. | HCl | 100-120 | -57,4 |
| 14 | $(CH_3)_2$-CH | 2 | 2 | O | H | H | L-menth. | HCl | Öl | -46,5 |
| 15 | $CH_3$ | 2 | 2 | O | 3,4-di-$CH_3O$- | | L-menth. | HCl | 110-130 | -42,5 |
| 16 | $(CH_3)_2$-CH | 2 | 2 | O | 3,4-di-$CH_3O$- | | L-menth. | HCl | Öl | -40,2 |
| 17 | $CH_3$ | 3 | 2 | O | H | H | L-menth. | HCl | 138-140 | -52,3 |
| 18 | H | 5 | 3 | O | H | H | cis-dihydronop. | HCl | 120-140 | -12,0 |
| 19 | H | 5 | 4 | O | H | H | cis-dihydronop. | HCl | 130-140 | -10,9 |
| 20 | $CH_3$ | 3 | 2 | O | H | H | cis-dihydronop. | Ba | Öl | -13,4 |
| 21 | $CH_3$ | 5 | 2 | O | H | H | cis-dihydronop. | HCl | Öl | -12,5 |
| 22 | $CH_3$ | 5 | 2 | O | 3,4-di-$CH_3O$- | | cis-dihydronop. | HCl | Öl | - 9,2 |
| 23 | H | 5 | 2 | O | 3,4-di-$CH_3O$- | | cis-dihydronop. | HCl | 130-140 | -11,2 |
| 24 | H | 4 | 2 | O | 2-$CH_3O$- | H | cis-dihydronop. | HCl | 130-135 | -11,5 |
| 25 | H | 5 | 2 | O | 2-$CH_3O$- | H | cis-dihydronop. | HCl | Öl | -11,2 |
| 26 | $CH_3$ | 2 | 2 | S | H | H | cis-dihydronop. | Ba | Öl | -17 |
| 27 | H | 5 | 2 | O | 2-Cl- | H | cis-dihydronop. | HCl | 110-120 | -11,3 |

| Bsp. Nr. | $R^1$ | n | m | Z | $R^2$ | $R^3$ | $R^4$ | Salz-form | phys. Konst. Fp., (Schmelzbereich) in °C | phys. Konst. $[\alpha]_D^{20}$ in ° (c=1, $CH_3OH$) |
|---|---|---|---|---|---|---|---|---|---|---|
| 28 | $CH_3$ | 3 | 2 | 0 | 3,4-di-$CH_3O$- | | cis-dihydronop. | HCl | 70- 80 | -10,2 |
| 29 | H | 4 | 1 | 0 | 3,4-di-Cl- | | cis-dihydronop. | HCl | 170-178 | -10,7 |
| 30 | H | 5 | 1 | 0 | 3-$CF_3$- | H | cis-dihydronop. | HCl | 86- 90 | -10,3 |
| 31 | H | 4 | 1 | 0 | 3-$CH_3$- | H | cis-dihydronop. | HCl | 130-140 | -12,7 |
| 32 | H | 4 | 1 | 0 | 3-F- | H | cis-dihydronop. | HCl | 130-135 | -11,2 |
| 33 | H | 4 | 1 | 0 | 3,4-O-$CH_2$-O- | | cis-dihydronop. | HCl | 178-180 | -11,2 |

HCl = Hydrochlorid, Ba = Base, Öl = ölig
cis-dihydronop. = cis-Dihydronopyl = 2-((1S,2S,5S)-6,6-Dimethylbicyclo[3.1.1]hept-2-yl)-ethyl
cis-myr. = cis-Myrtanyl = (1S,2S,5S)-6,6-Dimethylbicyclo[3.1.1]hept-2-yl)-methyl
trans-myr. = trans-Myrtanyl = (1S,2R,5S)-6,6-Dimethylbicyclo[3.1.1]hept-2-yl)-methyl
L-menth. = L-Menthyl = (1R,3R,4S)-1-Methyl-4-isopropylcyclohex-3-yl

Beispiel I:

Man stellte Tabletten in folgender Zusammensetzung pro Tablette her:

| | |
|---|---|
| N-(3-[2-((1S,2S,5S)-6,6-Dimethylbicyclo[3.1.1]hept-2-yl)-ethoxy]-propyl)-N-(phenethyl)-N-methylammonium-dihydrogenphosphat | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke und sodann zu Tabletten von 240 mg verpreßt. | 9 mg |

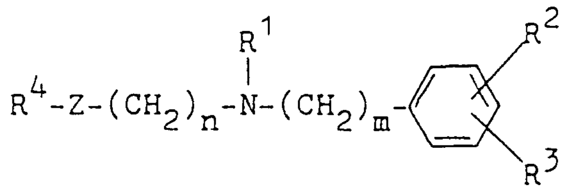 I

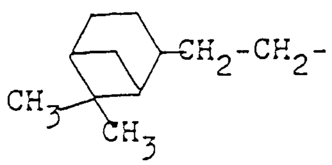

a

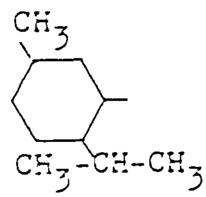

b

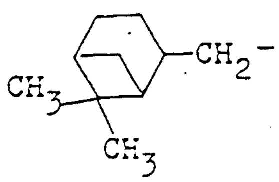

c

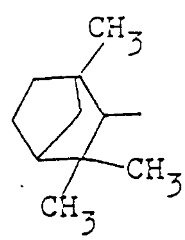

d

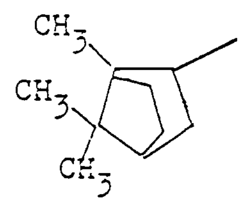

e

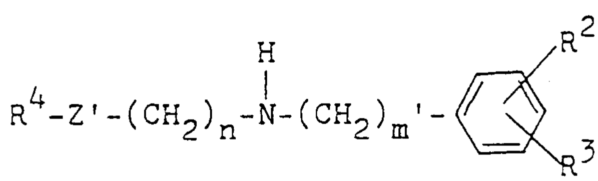 Ia

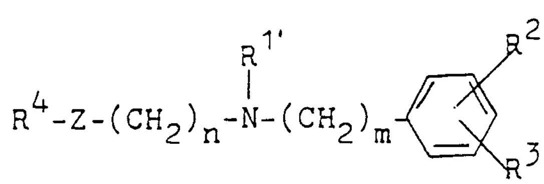 Ib

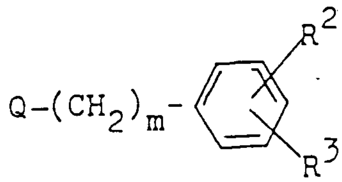

II

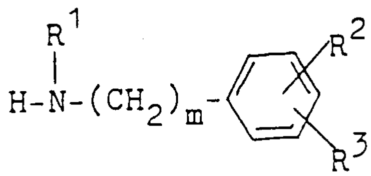

IIa

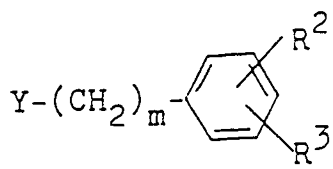

IIb

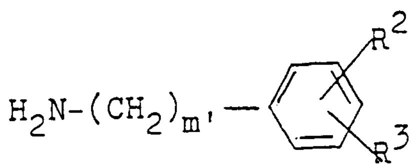

IIc

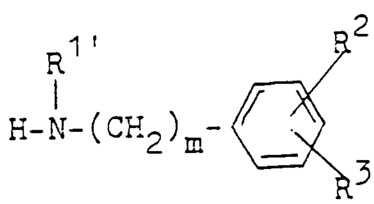

IId

$R^4$-Z-$(CH_2)_n$-Q' III

$R^4$-Z-$(CH_2)_n$-Y IIIa

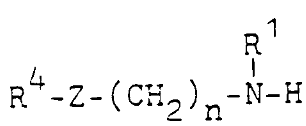

IIIb

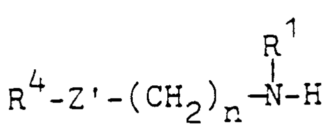

IIIc

$R^4$ Z'-$(CH_2)_n$-Y IIId

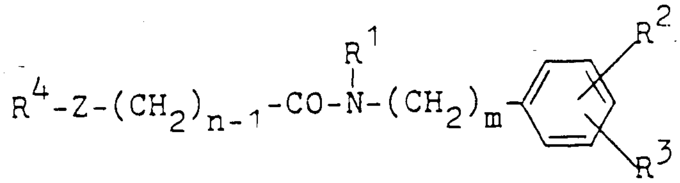

IV

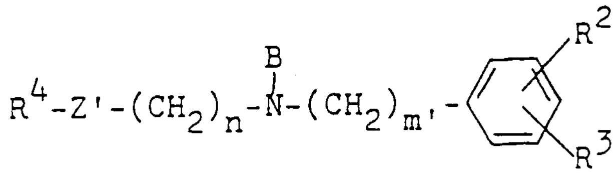

V

$R^4$-T VI

$R^4$-Z-H VIa

$R^4$-Y VIb

$R^4$-Z"-H VIc

$R^4$-OH VId

$R^4$-SH VIe

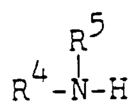

VIf

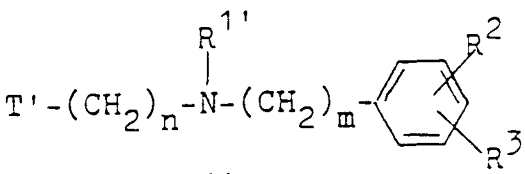

VII

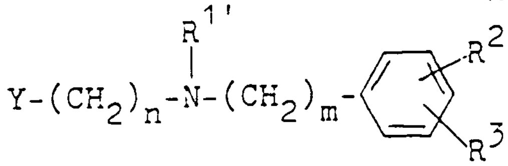

VIIa

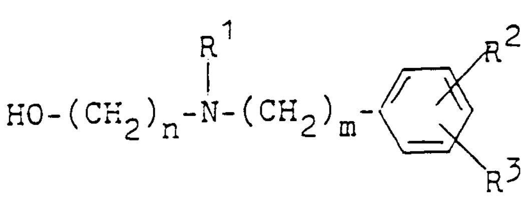

VIIb

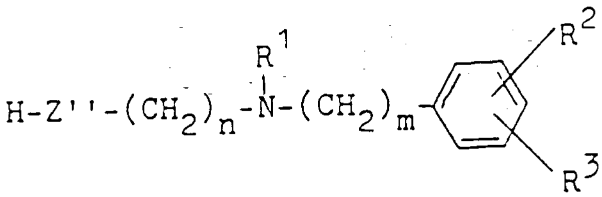

VIIc

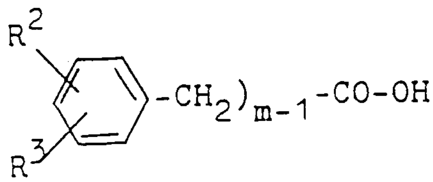

VIII

$R^1-NH_2$ IX

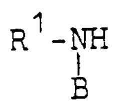

IX'

$B-NH_2$ IX"

$R^4-Z-(CH_2)_n-OH$ X

$HO-(CH_2)_n-OH$ XI

$X-(CH_2)_n-OH$ XII

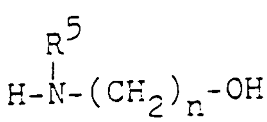

XIII

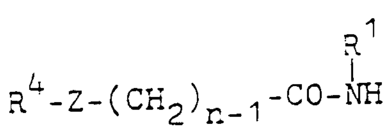

XIV

$R^4-Z-(CH_2)_{n-1}-CO-OH$ XV

$X-(CH_2)_{n-1}-CO-OH$ XVI

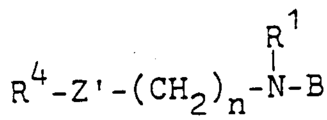

XVII

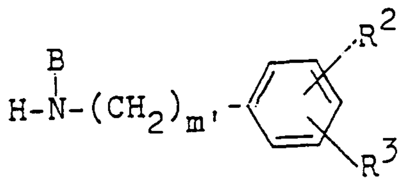

XVIII

$R^4$-S-CO-$CH_3$ XIX

$R^5$-$NH_2$ XX

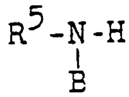

XX'

## Patentansprüche

### Patentansprüche für folgende Vertragsstaaten : AT, BE, CH, DE, DK, FR, GB, IT, LU, NL, SE

1. Verbindungen der allgemeinen Formel I

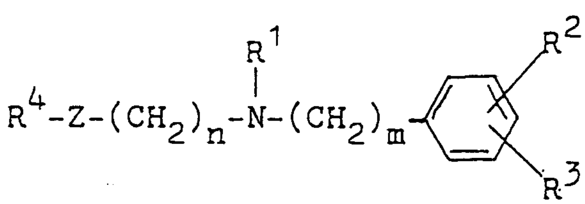

I

worin

| | |
|---|---|
| m | für 1-4 steht, |
| n | für 2-5 steht, |
| $R^1$ | Wasserstoff oder Alkyl mit 1-4 Kohlenstoffatomen bedeutet, |
| $R^2$ | Wasserstoff, Alkyl mit 1-4 Kohlenstoffatomen, Alkoxy mit 1-4 Kohlenstoffatomen, Halogen oder Trifluormethyl bedeutet, und |
| $R^3$ | Wasserstoff, Alkyl mit 1-4 Kohlenstoffatomen, Alkoxy mit 1-4 Kohlenstoffatomen oder Halogen bedeutet, oder |
| $R^2$ und $R^3$ | an benachbarte Kohlenstoffatome gebunden sind und gemeinsam eine Alkylendioxygruppe mit 1-2 Kohlenstoffatomen bedeuten, |
| $R^4$ | für einen gesättigten mono- oder bicyclischen Terpenkohlenwasserstoffrest mit 10 Kohlenstoffatomen oder für einen bicyclischen Kohlenwasserstoffrest mit 11 Kohlenstoffatomen mit der Formel a |

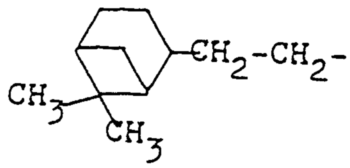

a

steht, und

Z für Sauerstoff, eine Gruppe N-$R^5$, worin $R^5$ Alkyl mit 1 - 4 Kohlenstoffatomen bedeutet, oder, falls $R^4$ eine Gruppe der Formel a bedeutet, auch für Schwefel steht,

und deren physiologisch verträgliche Säureadditionssalze.

**2.** Verbindungen nach Anspruch 1, dadurch gekennzeichnet, daß $R^4$ für einen mono- oder bicyclischen Kohlenwasserstoffrest mit 10 oder 11 Kohlenstoffatomen aus der Gruppe 2-(6,6-Dimethylbicyclo[3.1.1]hept-2-yl)-äthyl (= Dihydronopyl) der Formel a, 1-Methyl-4-isopropylcyclohex-3-yl (= Menthyl) der Formel b, 6,6-Dimethylbicyclo[3.1.1]hept-2-ylmethyl (= Myrtanyl) der Formel c, 1,3,3-Trimethylbicyclo[2.2.1]hept-2-yl (= Fenchyl) der Formel d und 1,7,7-Trimethylbicyclo[2.2.1]hept-2-yl (= Bornyl) der Formel e steht

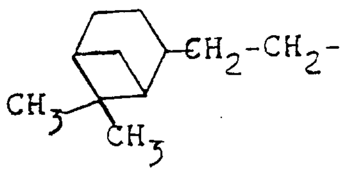

a

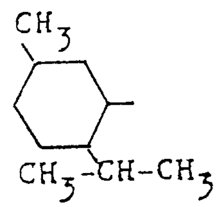

b

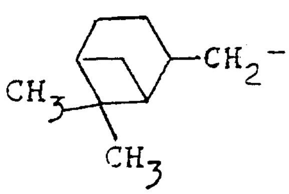

c

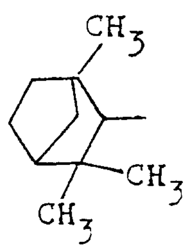

d

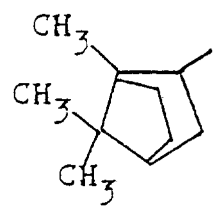

e

**3.** Verbindungen nach Anspruch 2, dadurch gekennzeichnet, daß $R^4$ für einen Rest der Formel a, b oder c steht.

**4.** Verbindungen nach Anspruch 3, dadurch gekennzeichnet, daß $R^4$ für einen 2-(6,6-Dimethylbicyclo[3.1.1]hept-2-yl)-äthylrest steht.

**5.** Verbindungen nach Anspruch 4, dadurch gekennzeichnet, daß der Rest $R^4$ überwiegend in der 1S,2S,5S-Konfiguration vorliegt.

**6.** Arzneimittel, enthaltend eine pharmakologisch wirksame Menge einer Verbindung gemäß Anspruch 1 und übliche pharmazeutische Hilfs- und Trägerstoffe.

**7.** Verfahren zur Herstellung von Verbindungen der allgemeinen Formel I

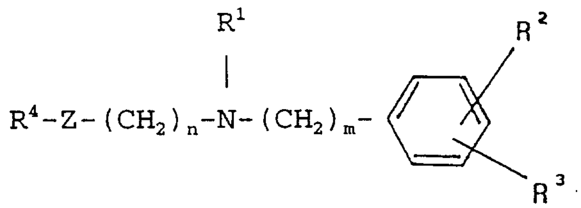

I

worin m, n, $R^1$, $R^2$, $R^3$, $R^4$ und 2 die in Anspruch 1 angegebene Bedeutung besitzen, und deren physiologisch verträglichen Säureadditionssalzen, dadurch gekennzeichnet, daß man

a) Verbindungen der allgemeinen Formel II

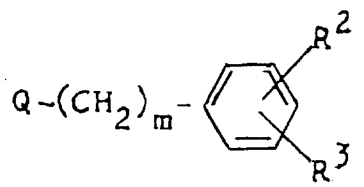

II

worin $R^2$, $R^3$ und m obige Bedeutung besitzen und Q für den Rest

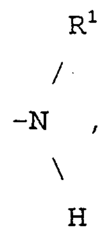

worin $R^1$ obige Bedeutung besitzt,
steht, oder falls m nicht 2 bedeutet, auch für eine abspaltbare Fluchtgruppe Y steht, umsetzt mit Verbindungen der allgemeinen Formel III

$$R^4Z\text{-}(CH_2)_n\text{-}Q' \qquad \text{III}$$

worin $R^4$, Z und n obige Bedeutung besitzen und Q' für eine abspaltbare Fluchtgruppe Y steht, falls Q den Rest

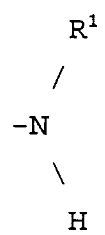

bedeutet, oder
Q' für den Rest

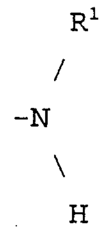

steht, falls Q eine abspaltbare Fluchtgruppe Y bedeutet, oder

b) Verbindungen der allgemeinen Formel IV

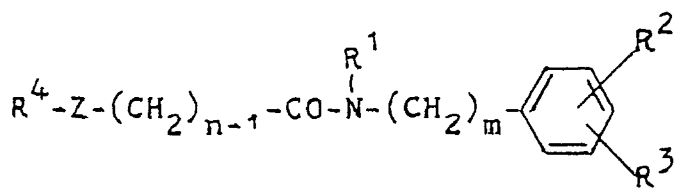

IV

worin $R^1$, $R^2$, $R^3$, $R^4$, Z, n und m obige Bedeutung besitzen, reduziert, oder

c) zur Herstellung von Verbindungen der allgemeinen Formel Ia

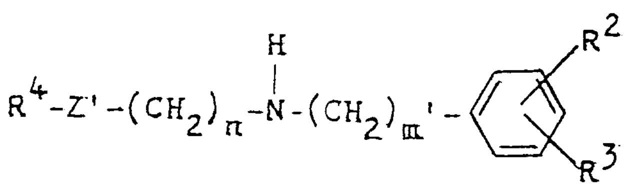

Ia

worin $R^2$, $R^3$, $R^4$ und n obige Bedeutung besitzen, m' 2-4 bedeutet und Z' die für Z angegebene Bedeutung mit Ausnahme von Schwefel besitzt, aus Verbindungen der allgemeinen Formel V

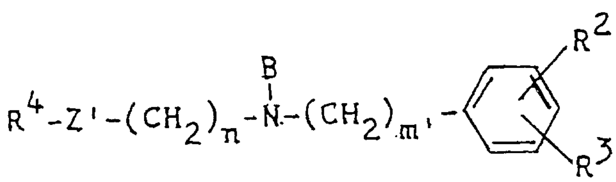

V

worin $R^2$, $R^3$, $R^4$, Z', n und m' obige Bedeutung besitzen, und B für eine hydrogenolytisch abspaltbare Gruppe steht, die Gruppe B hydrogenolytisch abspaltet, oder

d) zur Herstellung von Verbindungen der allgemeinen Formel Ib

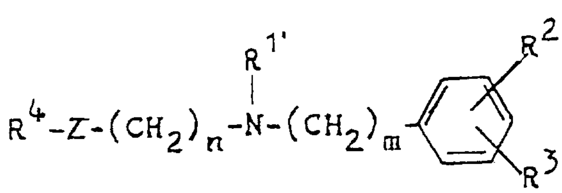

Ib

worin $R^2$, $R^3$, $R^4$, Z, n und m obige Bedeutung besitzen, und $R^{1'}$ Alkyl mit 1 - 4 Kohlenstoffatomen bedeutet, Verbindungen der allgemeinen Formel VI

$$R^4\text{-}T \qquad \text{VI}$$

worin $R^4$ obige Bedeutung besitzt und T für den Rest ZH, worin Z obige Bedeutung besitzt, oder für eine abspaltbare Fluchtgruppe Y, insbesondere Halogen, steht, umsetzt mit Verbindungen der allgemeinen Formel VII

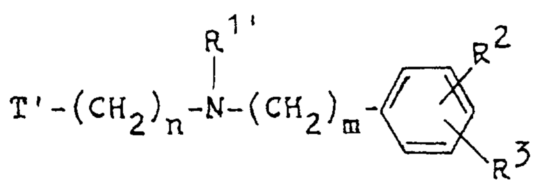

VII

worin $R^{1'}$, $R^2$, $R^3$, n und m obige Bedeutung besitzen, und T' für eine abspaltbare Fluchtgruppe Y, insbesondere Halogen, steht, falls T den Rest ZH bedeutet, oder T' für den Rest ZH steht, falls T eine abspaltbare Fluchtgruppe Y bedeutet,

und gewünschtenfalls erhaltene Verbindungen der allgemeinen Formel I, worin $R^1$ Wasserstoff bedeutet, zu entsprechenden Verbindungen der Formel I, worin $R^1$ Alkyl mit 1 - 4 Kohlenstoffatomen bedeutet, alkyliert, und gewünschtenfalls freie Verbindungen der Formel I in ihre Säureadditionssalze überführt oder die Säureadditionssalze in die freien Verbindungen der Formel I überführt.

**Patentansprüche für folgende Vertragsstaaten : ES, GR**

**1.** Verfahren zur Herstellung von Verbindungen der allgemeinen Formel I

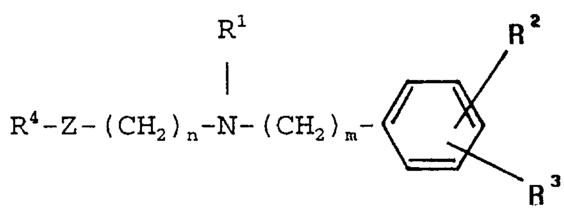

I

worin

| | |
|---|---|
| m | für 1-4 steht, |
| n | für 2-5 steht, |
| $R^1$ | Wasserstoff oder Alkyl mit 1 - 4 Kohlenstoffatomen bedeutet, |
| $R^2$ | Wasserstoff, Alkyl mit 1 - 4 Kohlenstoffatomen, Alkoxy mit 1 - 4 Kohlenstoffatomen, Halogen oder Trifluormethyl bedeutet, |
| $R^3$ | Wasserstoff, Alkyl mit 1 - 4 Kohlenstoffatomen, Alkoxy mit 1 - 4 Kohlenstoffatomen oder Halogen bedeutet, oder |
| $R^2$ und $R^3$ | an benachbarte Kohlenstoffatome gebunden sind und gemeinsam eine Alkylendioxygruppe mit 1-2 Kohlenstoffatomen bedeuten, |
| $R^4$ | für einen gesättigten mono- oder bicyclischen Terpenkohlenwasserstoffrest mit 10 Kohlenstoffatomen oder für einen bicyclischen Kohlenwasserstoffrest mit 11 Kohlenstoffatomen mit der Formel a |

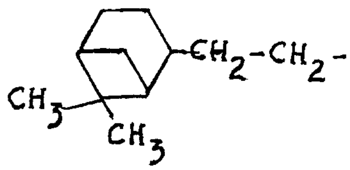

a

steht, und

Z für Sauerstoff, eine Gruppe N-$R^5$, worin $R^5$ Alkyl mit 1 - 4 Kohlenstoffatomen bedeutet, oder, falls $R^4$ eine Gruppe der Formel a bedeutet, auch für Schwefel steht,

und deren physiologisch verträglichen Säureadditionssalzen, dadurch gekennzeichnet, daß man

a) Verbindungen der allgemeinen Formel II

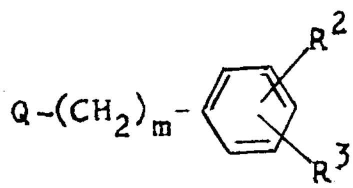

II

worin $R^2$, $R^3$ und m obige Bedeutung besitzen und Q für den Rest

```
  R1
 /
-N    ,
 \
  H
```

worin $R^1$ obige Bedeutung besitzt,
steht, oder falls m nicht 2 bedeutet, auch für eine abspaltbare Fluchtgruppe Y steht, umsetzt mit Verbindungen der allgemeinen Formel III

$$R^4Z\text{-}(CH_2)_n\text{-}Q' \qquad III$$

worin $R^4$, Z und n obige Bedeutung besitzen und Q' für eine abspaltbare Fluchtgruppe Y steht, falls Q den Rest

```
  R1
 /
-N
 \
  H
```

bedeutet, oder
Q' für den Rest

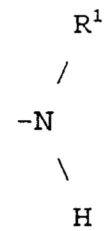

steht, falls Q eine abspaltbare Fluchtgruppe Y bedeutet, oder

b) Verbindungen der allgemeinen Formel IV

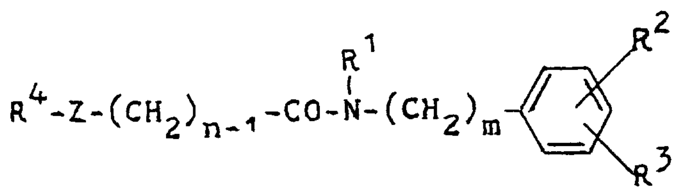

IV

worin $R^1$, $R^2$, $R^3$, $R^4$, Z, n und m obige Bedeutung besitzen, reduziert, oder

c) zur Herstellung von Verbindungen der allgemeinen Formel Ia

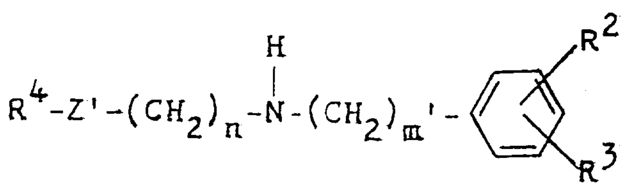

Ia

worin $R^2$, $R^3$, $R^4$ und n obige Bedeutung besitzen, m' 2-4 bedeutet und Z' die für Z angegebene Bedeutung mit Ausnahme von Schwefel besitzt, aus Verbindungen der allgemeinen Formel V

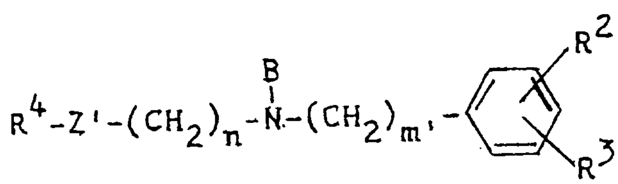

V

worin $R^2$, $R^3$, $R^4$, Z', n und m' obige Bedeutung besitzen, und B für eine hydrogenolytisch abspaltbare Gruppe steht, die Gruppe B hydrogenolytisch abspaltet, oder

d) zur Herstellung von Verbindungen der allgemeinen Formel Ib

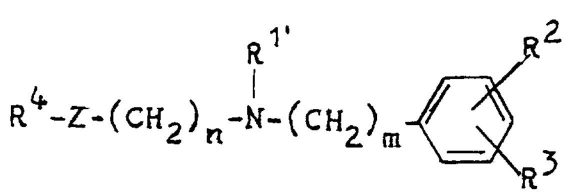

Ib

worin $R^2$, $R^3$, $R^4$, Z, n und m obige Bedeutung besitzen, und $R^{1'}$ Alkyl mit 1 - 4 Kohlenstoffatomen bedeutet, Verbindungen der allgemeinen Formel VI

$$R^4\text{-}T \qquad \text{VI}$$

worin $R^4$ obige Bedeutung besitzt und T für den Rest ZH, worin Z obige Bedeutung besitzt, oder für eine abspaltbare Fluchtgruppe Y, insbesondere Halogen, steht, umsetzt mit Verbindungen der allgemeinen Formel VII

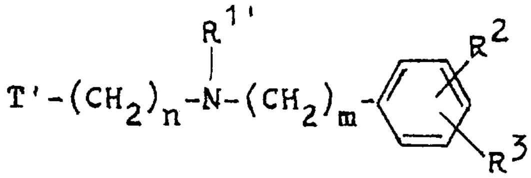

VII

worin $R^{1'}$, $R^2$, $R^3$, n und m obige Bedeutung besitzen, und T' für eine abspaltbare Fluchtgruppe Y, insbesondere Halogen, steht, falls T den Rest ZH bedeutet, oder T' für den Rest ZH steht, falls T eine abspaltbare Fluchtgruppe Y bedeutet,

und gewünschtenfalls erhaltene Verbindungen der allgemeinen Formel I, worin $R^1$ Wasserstoff bedeutet, zu entsprechenden Verbindungen der Formel I, worin $R^1$ Alkyl mit 1 - 4 Kohlenstoffatomen bedeutet, alkyliert, und gewünschtenfalls freie Verbindungen der Formel I in ihre Säureadditionssalze überführt oder die Säureadditionssalze in die freien Verbindungen der Formel I überführt.

2. Verfahren zur Herstellung von Verbindungen nach Anspruch 1, dadurch gekennzeichnet, daß Verbindungen hergestellt werden, worin $R^4$ für einen mono- oder bicyclischen Kohlenwasserstoffrest mit 10 oder 11 Kohlenstoffatomen aus der Gruppe 2-(6,6-Dimethylbicyclo[3.1.1]hept-2-yl)-äthyl (= Dihydronopyl) der Formel a, 1-Methyl-4-isopropylcyclohex-3-yl (= Menthyl) der Formel b, 6,6-Dimethylbicyclo[3.1.1]hept-2-ylmethyl (= Myrtanyl) der Formel c, 1,3,3-Trimethylbicyclo[2.2.1]hept-2-yl (= Fenchyl) der Formel d und 1,7,7-Trimethylbicyclo[2.2.1]hept-2-yl (= Bornyl) der Formel e steht

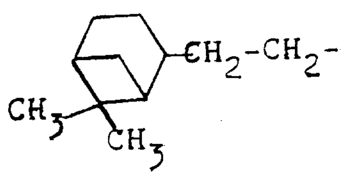

a

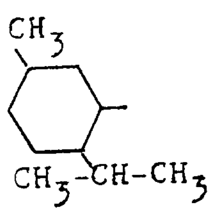

b

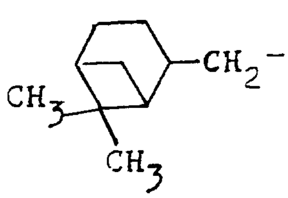

c

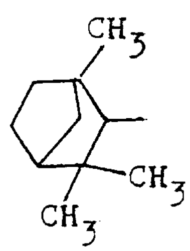

d

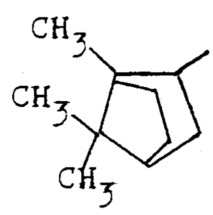

e

3. Verfahren nach Anspruch 2, dadurch gekennzeichnet, daß Verbindungen hergestellt werden, worin $R^4$ für einen Rest der Formel a, b oder c steht.

4. Verfahren nach Anspruch 3, dadurch gekennzeichnet, daß Verbindungen hergestellt werden, worin $R^4$ für einen 2-(6,6-Dimethylbicyclo[3.1.1]hept-2-yl)-äthylrest steht.

5. Verfahren nach Anspruch 4, dadurch gekennzeichnet, daß Verbindungen hergestellt werden, worin der Rest $R^4$ überwiegend in der 1S,2S,5S-Konfiguration vorliegt.

## Claims

### Claims for the following Contracting States : AT, BE, CH, DE, DK, FR, GB, IT, LU, NL, SE

1. Compounds of the general Formula I,

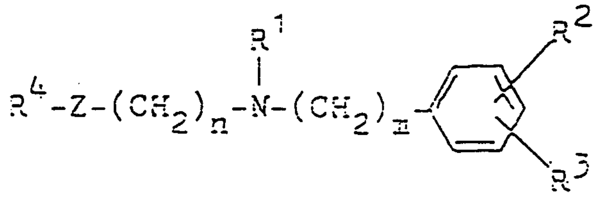

I

wherein

| | |
|---|---|
| m | stands for 1-4, |
| n | stands for 2-5, |
| $R^1$ | is hydrogen or alkyl with 1 - 4 carbon atoms, |
| $R^2$ | is hydrogen, alkyl with 1 - 4 carbon atoms, alkoxy with 1 - 4 carbon atoms, halogen or trifluoromethyl, and |
| $R^3$ | is hydrogen, alkyl with 1 - 4 carbon atoms, alkoxy with 1 - 4 carbon atoms or halogen, or |
| $R^2$ and $R^3$ | are linked to adjacent carbon atoms and together form an alkylenedioxy group with 1-2 carbon atoms, |
| $R^4$ | stands for a saturated monocyclic or bicyclic terpene hydrocarbon radical with 10 carbon atoms, or for a bicyclic hydrocarbon radical with 11 carbon atoms having the formula a, |

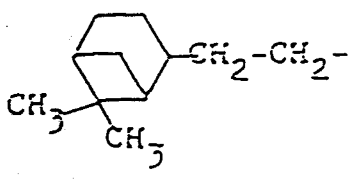

a

and

| | |
|---|---|
| Z | stands for oxygen, an N-$R^5$ group, wherein $R^5$ is alkyl with 1 - 4 carbon atoms, or, if $R^4$ is a group of Formula a, also for sulphur, |

and their physiologically compatible acid addition salts.

2. Compounds according to Claim 1, characterised in that $R^4$ stands for a monocyclic or bicyclic hydrocarbon radical with 10 or 11 carbon atoms from the group 2-(6,6-dimethylbicyclo[3.1.1]hept-2-yl)-ethyl (= dihydronopyl) of Formula a, 1-methyl-4-isopropylcyclohex-3-yl (= menthyl) of Formula b, 6,6-dimethylbicyclo[3.1.1]hept-2-ylmethyl (= myrtanyl) of Formula c, 1,3,3-trimethylbicyclo[2.2.1]hept-2-yl (= fenchyl) of Formula d and 1,7,7-trimethylbicyclo[2.2.1]hept-2-yl (= bornyl) of Formula e.

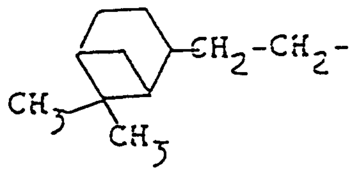

a

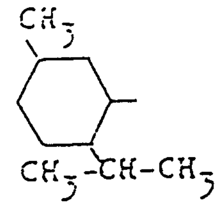

b

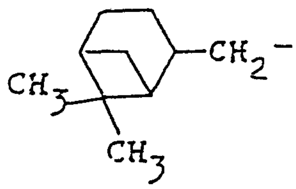

c

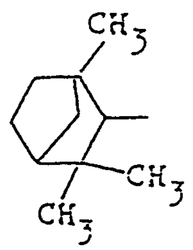

d

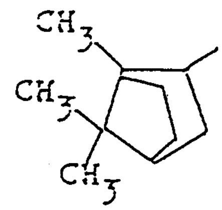

e

3. Compounds according to Claim 2, characterised in that $R^4$ stands for a radical of Formula a, b or c.

4. Compounds according to Claim 3, characterised in that $R^4$ stands for a 2-(6,6-dimethylbicyclo[3.1.1]hept-2-yl)-ethyl radical.

5. Compounds according to Claim 4, characterised in that the radical $R^4$ is predominantly present in the 1S,2S,5S-configuration.

6. Medicament, containing a pharmacologically active quantity of a compound according to Claim 1 and conventional pharmaceutical auxiliaries and carriers.

7. Method for preparing compounds of the general Formula I,

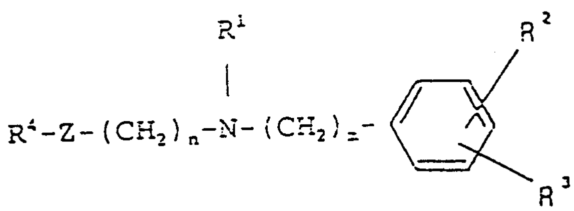

I

wherein m, n, $R^1$, $R^2$, $R^3$, $R^4$ and Z have the meanings given in Claim 1, and their physiologically compatible acid addition salts, characterised in that

a) compounds of the general Formula II,

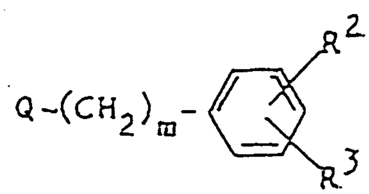

II

wherein $R^2$, $R^3$ and m have the above meanings and Q stands for the radical

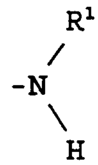 ,

wherein $R^1$ has the above meaning, or if m is not 2, also stands for a cleavable leaving group Y, are reacted with compounds of the general Formula III,

$$R^4Z\text{-}(CH_2)_n\text{-}Q' \qquad \text{III}$$

wherein $R^4$, Z and n have the above meanings and Q' stands for a cleavable leaving group Y, if Q stands for the radical

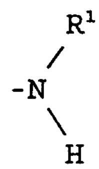 ,

or
Q' stands for the radical

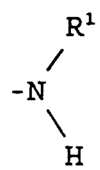 ,

if Q is a cleavable leaving group Y, or

b) compounds of the general Formula IV,

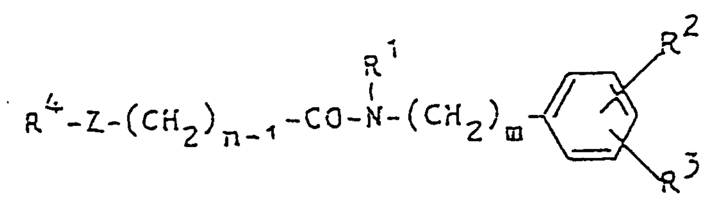

IV

wherein $R^1$, $R^2$, $R^3$, $R^4$, Z, n and m have the above meanings, are reduced, or

c) to prepare compounds of the general Formula Ia,

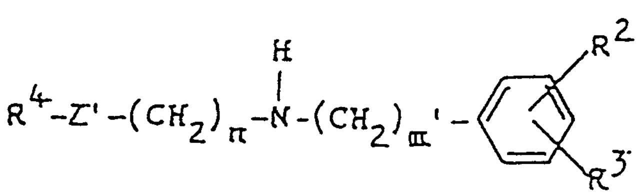

Ia

wherein $R^2$, $R^3$, $R^4$ and n have the above meanings, m' is 2-4 and Z' has the meaning given for Z with the exception of sulphur, the group B is hydrogenolytically split from compounds of the general Formula V,

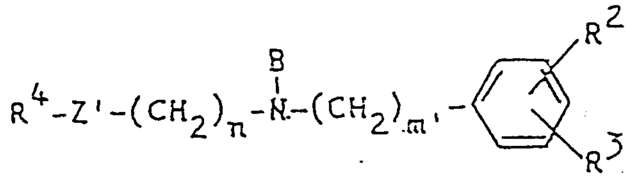

V

wherein $R^2$, $R^3$, $R^4$, Z', n and m' have the above meanings, and B stands for a hydrogenolytically cleavable group, or

d) to prepare compounds of the general Formula Ib,

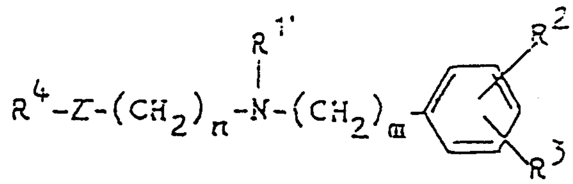

Ib

wherein $R^2$, $R^3$, $R^4$, Z, n and m have the above meanings, and $R^{1'}$ is alkyl with 1 - 4 carbon atoms, compounds of the general Formula VI,

$$R^4\text{-}T \qquad \text{VI}$$

wherein $R^4$ has the above meaning and T stands for the radical ZH, wherein Z has the above meaning, or for a cleavable leaving group Y, in particular halogen, are reacted with compounds of the general Formula VII,

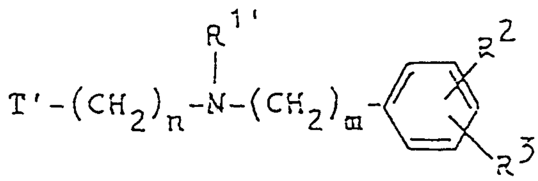

VII

wherein $R^{1'}$, $R^2$, $R^3$, n and m have the above meanings, and T' stands for a cleavable leaving group Y, in particular halogen, if T is the radical ZH, or T' stands for the radical ZH if T is a cleavable leaving group Y,

and if desired resulting compounds of the general Formula I wherein $R^1$ is hydrogen are alkylated to give corresponding compounds of Formula I wherein $R^1$ is alkyl with 1 - 4 carbon atoms, and if desired free compounds of Formula I are converted into their acid addition salts or the acid addition salts are converted into the free compounds of Formula I.

**Claims for the following Contracting States : ES, GR**

1. Method for preparing compounds of the general Formula I,

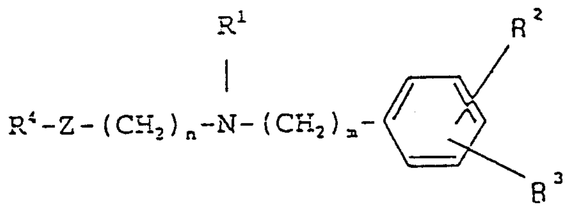

I

wherein

| | |
|---|---|
| m | stands for 1-4, |
| n | stands for 2-5, |
| $R^1$ | is hydrogen or alkyl with 1 - 4 carbon atoms, |
| $R^2$ | is hydrogen, alkyl with 1 - 4 carbon atoms, alkoxy with 1 - 4 carbon atoms, halogen or trifluoromethyl, and |
| $R^3$ | is hydrogen, alkyl with 1 - 4 carbon atoms, alkoxy with 1 - 4 carbon atoms or halogen, or |
| $R^2$ and $R^3$ | are linked to adjacent carbon atoms and together form an alkylenedioxy group with 1-2 carbon atoms, |
| $R^4$ | stands for a saturated monocyclic or bicyclic terpene hydrocarbon radical with 10 carbon atoms, or for a bicyclic hydrocarbon radical with 11 carbon atoms having the formula a, |

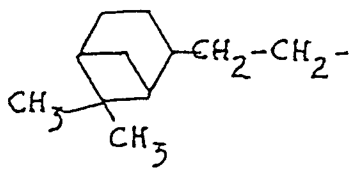

a

and

| | |
|---|---|
| Z | stands for oxygen, an N-$R^5$ group, wherein $R^5$ is alkyl with 1 - 4 carbon atoms, or, if $R^4$ is a group of Formula a, also for sulphur, |

and their physiologically compatible acid addition salts, characterised in that

a) compounds of the general Formula II,

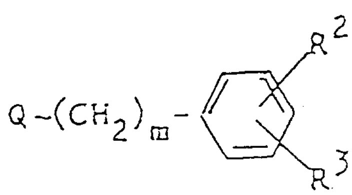

II

wherein $R^2$, $R^3$ and m have the above meanings and Q stands for the radical

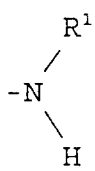

,

wherein $R^1$ has the above meaning, or if m is not 2, also stands for a cleavable leaving group Y, are reacted with compounds of the general Formula III,

$$R^4Z\text{-}(CH_2)_n\text{-}Q' \qquad III$$

wherein $R^4$, Z and n have the above meanings and Q' stands for a cleavable leaving group Y, if Q stands for the radical

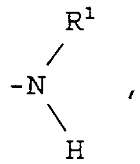

or
Q' stands for the radical

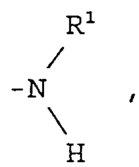

if Q is a cleavable leaving group Y, or

b) compounds of the general Formula IV,

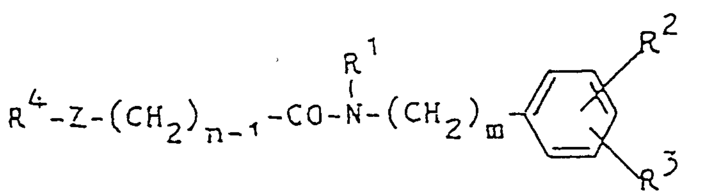 IV

wherein $R^1$, $R^2$, $R^3$, $R^4$, Z, n and m have the above meanings, are reduced, or

c) to prepare compounds of the general Formula Ia,

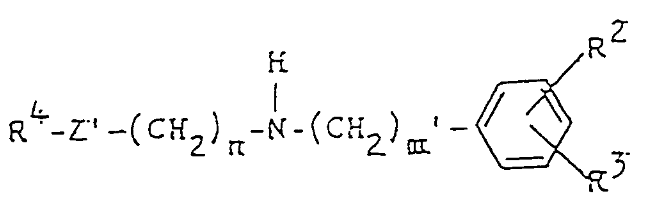 Ia

wherein $R^2$, $R^3$, $R^4$ and n have the above meanings, m' is 2-4 and Z' has the meaning given for Z with the exception of sulphur, the group B is hydrogenolytically split from compounds of the general Formula V,

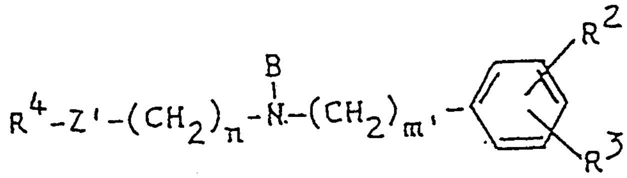

V

wherein $R^2$, $R^3$, $R^4$, Z', n and m' have the above meanings, and B stands for a hydrogenolytically cleavable group, or

d) to prepare compounds of the general Formula Ib,

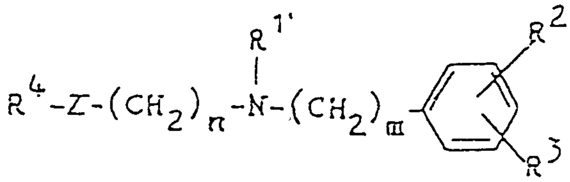

Ib

wherein $R^2$, $R^3$, $R^4$, Z, n and m have the above meanings, and $R^{1'}$ is alkyl with 1 - 4 carbon atoms, compounds of the general Formula VI,

$$R^4\text{-}T \qquad \text{VI}$$

wherein $R^4$ has the above meaning and T stands for the radical ZH, wherein Z has the above meaning, or for a cleavable leaving group Y, in particular halogen, are reacted with compounds of the general Formula VII,

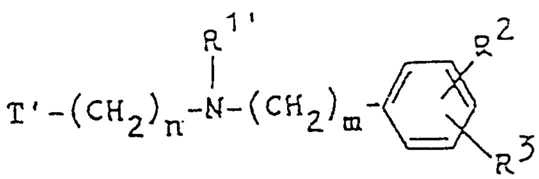

VII

wherein $R^{1'}$, $R^2$, $R^3$, n and m have the above meanings, and T' stands for a cleavable leaving group Y, in particular halogen, if T is the radical ZH, or T' stands for the radical ZH if T is a cleavable leaving group Y,

and if desired resulting compounds of the general Formula I wherein $R^1$ is hydrogen are alkylated to give corresponding compounds of Formula I wherein $R^1$ is alkyl with 1 - 4 carbon atoms, and if desired free compounds of Formula I are converted into their acid addition salts or the acid addition salts are converted into the free compounds of Formula I.

2. Method for preparing compounds according to Claim 1, characterised in that compounds are produced in which $R^4$ stands for a monocyclic or bicyclic hydrocarbon radical with 10 or 11 carbon atoms from the group 2-(6,6-dimethylbicyclo[3.1.1]hept-2-yl)-ethyl (= dihydronopyl) of Formula a, 1-methyl-4-isopropylcyclohex-3-yl (= menthyl) of Formula b, 6,6-dimethylbicyclo[3.1.1]hept-2-ylmethyl (= myrtanyl) of Formula c, 1,3,3-trimethylbicyclo[2.2.1]hept-2-yl (= fenchyl) of Formula d and 1,7,7-trimethylbicyclo[2.2.1]hept-2-yl (= bornyl) of Formula e.

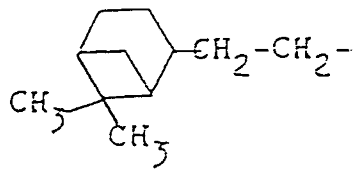

a

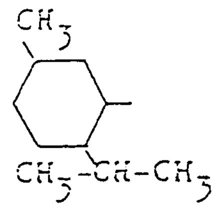

b

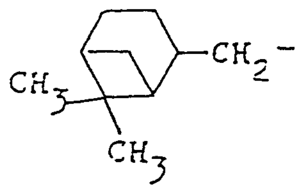

c

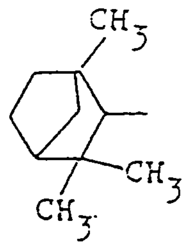

d

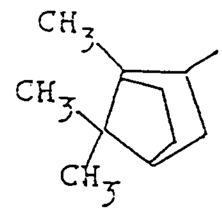

e

3. Method according to Claim 2, characterised in that compounds are produced in which $R^4$ stands for a radical of Formula a, b or c.

4. Method according to Claim 3, characterised in that compounds are produced in which $R^4$ stands for a 2-(6,6-dimethylbicyclo[3.1.1]hept-2-yl)-ethyl radical.

5. Method according to Claim 4, characterised in that compounds are produced in which the radical $R^4$ is predominantly present in the 1S,2S,5S-configuration.

**Revendications**

**Revendications pour les Etats contractants suivants : AT, BE, CH, DE, DK, FR, GB, IT, LU, NL, SE**

1. Composés de la formule générale I

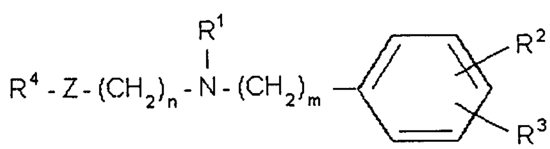

I

dans laquelle

| | |
|---|---|
| m | représente 1 à 4, |
| n | représente 2 à 5, |
| $R^1$ | signifie l'hydrogène ou un alkyle avec 1 à 4 atomes de carbone, |
| $R^2$ | signifie l'hydrogène, un alkyle avec 1 à 4 atomes de carbone, un groupe alkoxy avec 1 à 4 atomes de carbone, un halogène ou le groupe trifluorométhyle et |
| $R^3$ | signifie l'hydrogène, un alkyle avec 1 à 4 atomes de carbone, un groupe alkoxy avec 1 à 4 atomes de carbone ou un halogène, ou |
| $R^2$ et $R^3$ | sont liés à des atomes de carbone contigus et signifient ensemble un groupe alkylènedioxy avec 1 à 2 atomes de carbone, |
| $R^4$ | représente un reste d'hydrocarbure terpénique monocyclique ou bicyclique saturé avec 10 atomes de carbone ou un reste d'hydrocarbure bicyclique avec 11 atomes de carbone de la formule a |

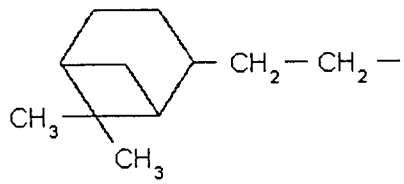

a

et

Z représente l'oxygène, un groupe N-$R^5$, $R^5$ signifiant un alkyle avec 1 à 4 atomes de carbone ou, au cas où $R^4$ signifie un groupe de la formule a, également le soufre

et leurs sels d'addition d'acide compatibles physiologiquement.

2. Composés selon la revendication 1, caractérisés en ce que $R^4$ représente un reste d'hydrocarbure monocyclique ou bicyclique avec 10 ou 11 atomes de carbone du groupe 2-(6,6-diméthylbicyclo[3.1.1]hept-2-yl)-éthyle (= dihydronopyle) de la formule a, 1-méthyl-4-isopropylcyclohex-3-yle (= menthyle) de la formule b, 6,6-diméthylbicyclo[3.1.1]hept-2-ylméthyle (= myrtanyle) de la formule c, 1,3,3-triméthylbicyclo[2.2.1]hept-2-yle (= fenchyle) de la formule d et 1,7,7-triméthylbicyclo[2.2.1]hept-2-yle (= bornyle)de la formule e.

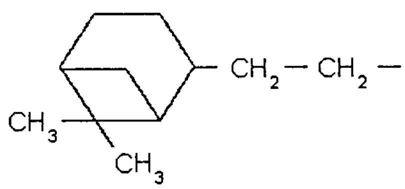

a

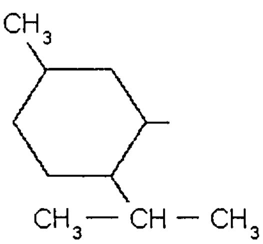

b

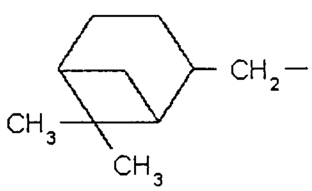

c

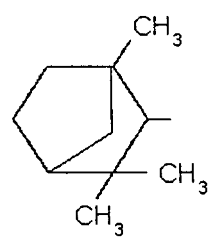

d

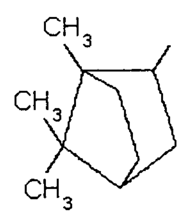

e

3. Composés selon la revendication 2, caractérisés en ce que $R^4$ représente un reste de la formule a, b ou c.

4. Composés selon la revendication 3, caractérisés en ce que $R^4$ représente un reste 2-(6,6-diméthylbicyclo[3.1.1]hept-2-yl)-éthyle.

5. Composés selon la revendication 4, caractérisés en ce que le reste $R^4$ existe de manière prépondérante dans la configuration 1S, 2S, 5S.

6. Médicament, contenant une quantité pharmacologiquement efficace d'un composé selon la revendication 1 et des substances auxiliaires et porteuses pharmaceutiques usuelles.

**7.** Procédé pour la préparation de composés de la formule générale I

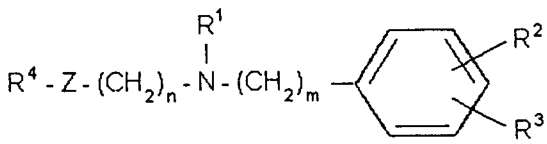

I

dans laquelle m, n, $R^1$, $R^2$, $R^3$, $R^4$ et Z possèdent la signification indiquée dans la revendication 1 et de leurs sels d'addition d'acide physiologiquement compatibles, caractérisé en ce que

a) on fait réagir des composés de la formule générale II

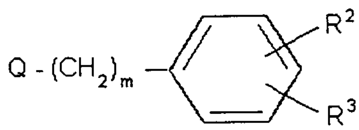

II

dans laquelle $R^2$, $R^3$ et m possèdent la signification ci-dessus et Q représente le reste

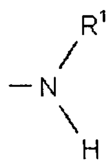

dans lequel $R^1$ possède la signification ci-dessus, ou au cas où m ne signifie pas 2, représente aussi un groupe partant éliminable Y, avec des composés de la formule générale III

$$R^4\text{-}Z\text{-}(CH_2)_n\text{-}Q' \qquad \text{III}$$

dans laquelle $R^4$, Z et n possèdent la signification ci-dessus et Q' représente un groupe partant éliminable Y, au cas où Q représente le reste

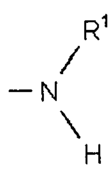

ou Q' représente le reste

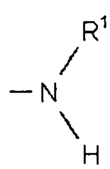

,

au cas où Q signifie un groupe partant éliminable Y ou

b) on réduit des composés de la formule générale IV

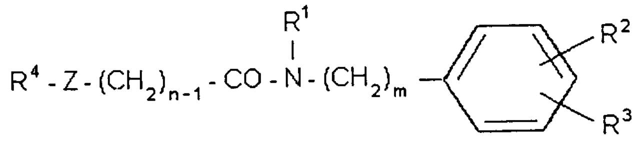

IV

dans laquelle $R^1$, $R^2$, $R^3$, $R^4$, Z, n et m possèdent la signification ci-dessus ou

c) pour préparer des composés de la formule générale Ia

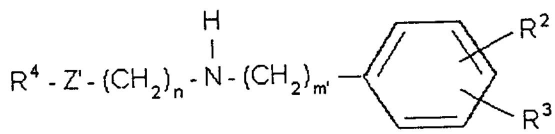

Ia

dans laquelle $R^2$, $R^3$, $R^4$ et n possèdent la signification ci-dessus, m' signifie 2 à 4 et Z' possède la signification indiquée pour Z à l'exception du soufre, on élimine, de composés de la formule générale V

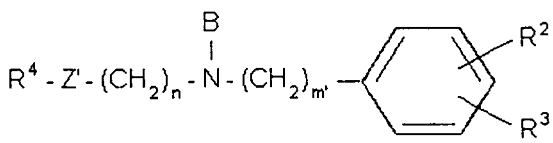

V

dans laquelle $R^2$, $R^3$, $R^4$, Z', n et m' possèdent la signification ci-dessus et B représente un groupe éliminable par hydrogénolyse, le groupe B par hydrogénolyse, ou

d) pour préparer des composés de la formule générale Ib

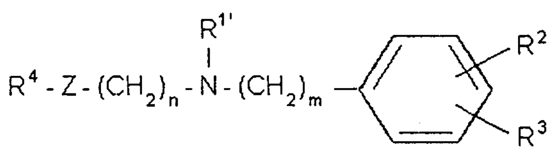

Ib

dans laquelle $R^2$, $R^3$, $R^4$, Z, n et m possèdent la signification ci-dessus et $R^{1'}$ représente un alkyle avec 1 à 4 atomes de carbone, on fait réagir des composés de la formule générale VI

$R^4$-T VI

dans laquelle $R^4$ possède la signification ci-dessus et T représente le reste ZH, dans lequel Z possède la signification ci-dessus, ou représente un groupe partant éliminable Y, en particulier un halogène, avec des composés de la formule générale VII

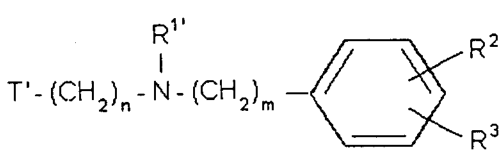

VII

dans laquelle $R^{1'}$, $R^2$, $R^3$, n et m possèdent la signification ci-dessus et T' représente un groupe partant éliminable Y, en particulier un halogène, au cas où T signifie le reste ZH, ou T' représente le reste ZH, au cas où T signifie un groupe partant éliminable Y,

et, au cas où on le souhaite, on alkyle des composés obtenus de la formule générale I, dans laquelle $R^1$ signifie l'hydrogène, en composés correspondants de la formule I, dans laquelle $R^1$ signifie un alkyle avec 1 à 4 atomes de carbone, et, au cas où on le souhaite, on transforme les composés libres de la formule I en leurs sels d'addition d'acide ou les sels d'addition d'acide en composés libres de la formule I.

**Revendications pour les Etats contractants suivants : ES, GR**

**1.** Procédé pour la préparation de composés de la formule générale I

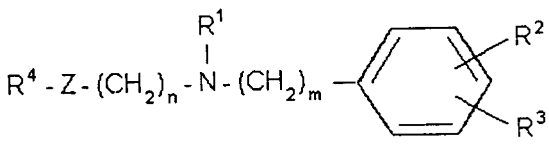

I

dans laquelle

| | |
|---|---|
| m | représente 1 à 4, |
| n | représente 2 à 5, |
| $R^1$ | signifie l'hydrogène ou un alkyle avec 1 à 4 atomes de carbone, |
| $R^2$ | signifie l'hydrogène, un alkyle avec 1 à 4 atomes de carbone, un groupe alkoxy avec 1 à 4 atomes de carbone, un halogène ou le groupe trifluorométhyle, |
| $R^3$ | signifie l'hydrogène, un alkyle avec 1 à 4 atomes de carbone, un groupe alkoxy avec 1 à 4 atomes de carbone ou un halogène, ou |
| $R^2$ et $R^3$ | sont liés à des atomes de carbone contigus et signifient ensemble un groupe alkylènedioxy avec 1 à 2 atomes de carbone, |
| $R^4$ | représente un reste d'hydrocarbure terpénique monocyclique ou bicyclique saturé avec 10 atomes de carbone ou un reste d'hydrocarbure bicyclique avec 11 atomes de carbone de la formule a |

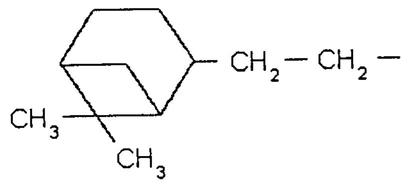

a

et

| | |
|---|---|
| Z | représente l'oxygène, un groupe $N-R^5$, $R^5$ signifiant un alkyle avec 1 à 4 atomes de carbone ou, au cas où $R^4$ signifie un groupe de la formule a, également le soufre et leurs sels d'addition d'acide compatibles physiologiquement, caractérisé en ce que |

a) l'on fait réagir des composés de la formule générale II

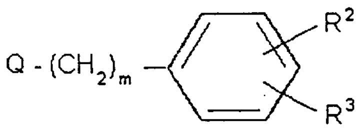

II

dans laquelle $R^2$, $R^3$ et m possèdent la signification ci-dessus et Q représente le reste

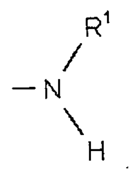

dans lequel $R^1$ possède la signification ci-dessus, ou au cas où m ne signifie pas 2, représente aussi un groupe partant éliminable Y, avec des composés de la formule générale III

$$R^4\text{-}Z\text{-}(CH_2)_n\text{-}Q' \qquad \text{III}$$

dans laquelle $R^4$, Z et n possèdent la signification ci-dessus et Q' représente un groupe partant éliminable Y, au cas où Q représente le reste

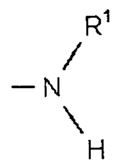

ou Q' représente le reste

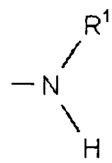 ,

au cas où Q signifie un groupe partant éliminable Y, ou

b) l'on réduit des composés de la formule générale IV

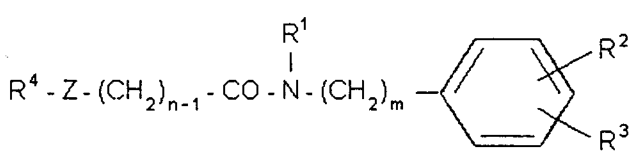

IV

dans laquelle $R^1$, $R^2$, $R^3$, $R^4$, Z, n et m possèdent la signification ci-dessus, ou

c) pour préparer des composés de la formule générale la

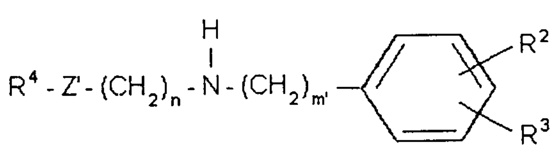

Ia

dans laquelle $R^2$, $R^3$, $R^4$ et n possèdent la signification ci-dessus, m' signifie 2-4 et Z' possède la signification indiquée pour Z à l'exception du soufre, on élimine, de composés de la formule générale V

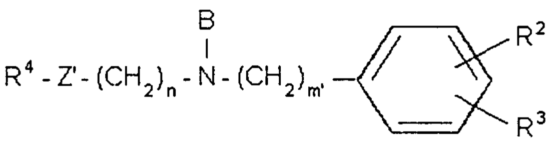

V

dans laquelle $R^2$, $R^3$, $R^4$, Z', n et m' possèdent la signification ci-dessus et B représente un groupe éliminable par hydrogénolyse, le groupe B par hydrogénolyse, ou

d) pour préparer des composés de la formule générale Ib

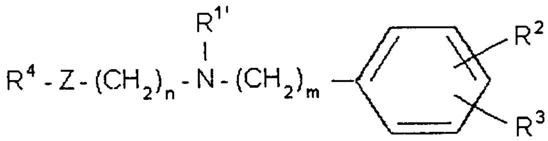

Ib

dans laquelle $R^2$, $R^3$, $R^4$, Z, n et m possèdent la signification ci-dessus et $R^{1'}$ représente un alkyle avec 1 à 4 atomes de carbone, on fait réagir des composés de la formule générale VI

$R^4$-T VI

dans laquelle $R^4$ possède la signification ci-dessus et T représente le reste ZH, dans lequel Z possède la signification ci-dessus, ou représente un groupe partant éliminable Y, en particulier un halogène, avec des composés de la formule générale VII

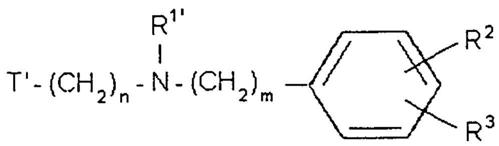

VII

dans laquelle $R^{1'}$, $R^2$, $R^3$, n et m possèdent la signification ci-dessus et T' représente un groupe partant éliminable Y, en particulier un halogène, au cas où T signifie le reste ZH, ou T' représente le reste ZH, au cas où T signifie un groupe partant éliminable Y,

et, au cas où on le souhaite, on alkyle des composés obtenus de la formule générale I, dans laquelle $R^1$ signifie l'hydrogène, en composés correspondants de la formule I, dans laquelle $R^1$ signifie un alkyle avec 1 à 4 atomes de carbone, et, au cas où on le souhaite, on transforme les composés libres de la formule I en leurs sels d'addition d'acide ou les sels d'addition d'acide en composés libres de la formule I.

2. Procédé pour la préparation de composés selon la revendication 1, caractérisé en ce que l'on prépare des composés, dans lesquels $R^4$ représente un reste d'hydrocarbure monocyclique ou bicyclique avec 10 ou 11 atomes de carbone du groupe 2-(6,6-diméthylbicyclo[3.1.1]hept-2-yl)-éthyle (= dihydronopyle) de la formule a, 1-méthyl-4-isopropylcyclohex-3-yl (= menthyle) de la formule b, 6,6-diméthylbicyclo[3.1.1]hept-2-ylméthyle (= myrtanyle) de la formule c, 1,3,3-triméthylbicyclo[2.2.1]hept-2-yl (= fenchyle) de la formule d et 1,7,7-triméthylbicyclo[2.2.1]hept-2-yl (= bornyle) de la formule e.

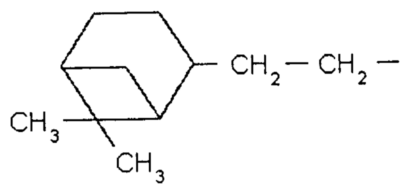

a

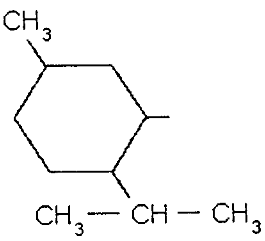

b

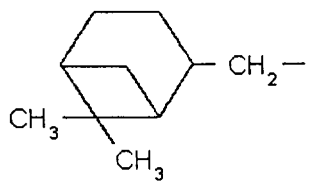

c

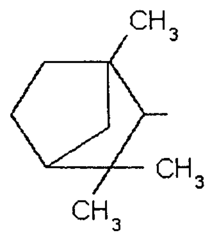

d

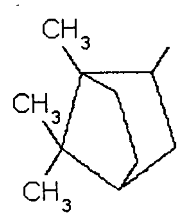

e

**3.** Procédé selon la revendication 2, caractérisé en ce que l'on prépare des composés, dans lesquels $R^4$ représente un reste de la formule a, b ou c.

**4.** Procédé selon la revendication 3, caractérisé en ce que l'on prépare des composés, dans lesquels $R^4$ représente un reste 2-(6,6-diméthylbicyclo[3.1.1]hept-2-yl)éthyle.

**5.** Procédé selon la revendication 4, caractérisé en ce que l'on prépare des composés dans lesquels $R^4$ se trouve de manière prépondérante en configuration 1S, 2S, 5S.